# EUROPEAN PATENT APPLICATION

(11) **EP 4 516 309 A1**
(43) Date of publication of application: **05.03.2025**
(21) Application number: 23382891.2
(22) Date of filing: 01.09.2023
(51) Int. Cl.: A61K 38/22, A61K 38/02, A61P 25/00, A61K 31/00

(54) **CRHR1 AGONISTS FOR USE IN THE TREATMENT OF SOCIAL ANXIETY DISORDERS**

(71) Applicant: Consejo Superior de Investigaciones Cientificas, 28006 Madrid (ES)
(72) Inventor: Leroy, Felix Raymond Michel, Alicante (ES); De Leon Reyes, Noelia Sofía, Alicante (ES)
(74) Representative: Pons

(57) **Abstract**

The invention relates to the use of a CRHR1 agonist, or composition comprising it, in the treatment and/or prevention of social anxiety disorders such as separation anxiety or avoidant personality disorders, particularly the use of the corticotropin-releasing hormone (CRH) as CRHR1 agonist.

## Description

The invention relates to the use of a CRHR1 agonist, or composition comprising it, in the treatment and/or prevention of social anxiety disorders such as separation anxiety or avoidant personality disorders, particularly the use of the corticotropin-releasing hormone (CRH) as CRHR1 agonist.

### BACKGROUND ART

Social preference, the decision to interact with one conspecific over another, is a feature displayed by gregarious animals, which is critical to navigate their social space. Adult rodents prefer to interact with their kin, individuals from specific strains and members of the opposite sex. In addition to innate factors (e.g., kin, strain, and sex), social preference is also influenced by social memory, social hierarchy and the affective state of the conspecific. Thus, adult rodents display social novelty preference (SNP), choosing to interact with novel individuals over familiar ones (Amdam, G. V., and Hovland, A.L. (2012). Measuring Animal Preferences and Choice Behavior. Nature Education Knowledge 3, 1-9.).

For the last two decades, social novelty preference has been used as a proxy to assess social memory but the neuronal circuits mediating social novelty preference remain elusive (Okuyama T, Kitamura T, Roy DS, Itohara S, Tonegawa S. Ventral CA1 neurons store social memory. Science. 2016 Sep 30;353(6307):1536-1541). In particular, it is unknown whether social novelty preference is due solely to a rewarding signal for novel social interactions or also involves the suppression of exploration of familiar individuals.

Memory-based preferences, such as social novelty preference, also have a developmental window and can change during the life of altricial animals. For example, young mice prefer their mother to unfamiliar dams until weaning when they begin to prefer unfamiliar dams over their mother (Laham, B.J., Diethorn, E.J., and Gould, E. (2021). Newborn mice form lasting CA2-dependent memories of their mothers. Cell Rep. 34, 108668.). Similarly, rat pups display a preference for their familiar siblings during the first 2 postnatal weeks, after which the preference shifts toward novel pups (Hepper, P.G. (1983). Sibling recognition in the rat. Anim. Behav. 31, 1177-1191). Although the mechanisms that regulate these developmental shifts remain elusive, the lateral septum (LS), a brain region associated with the regulation of motivated behaviors including social interactions, is necessary for kinship/familiarity preference in young rats as well as for social novelty preference in adult rodents (Menon, R., Süß, T., Oliveira, V.E. de M., Neumann, I.D., and Bludau, A. (2022). Neurobiology of the lateral septum: regulation of social behavior. Trends Neurosci. 45, 27-40 and Clemens, A.M., Wang, H., and Brecht, M. (2020). The lateral septum mediates kinship behavior in the rat. Nat. Commun. 11, 3161). Moreover, the ventral aspect of medial prefrontal cortex (mPFC), the infra-limbic area (ILA), is known for its involvement in decision-making, responds to social stimuli and is also necessary for social novelty preference (Yizhar, O., and Levy, D.R. (2021). The social dilemma: prefrontal control of mammalian sociability. Curr. Opin. Neurobiol. 68, 67-75). The mPFC projects to LS but how these regions integrate social memory cues and communicate to regulate social interactions is still unclear.

### DESCRIPTION OF THE INVENTION

The inventors demonstrate through a combination of electrophysiological, chemogenetic, optogenetic, calcium recording and gene silencing techniques that the release of CRH from ILA neurons (ILA^{CRH} neurons) into the rostral region of LS (rLS) suppresses social interaction with familiar mice. This circuit therefore regulates familiarization (decrease in interaction as a novel rodent becomes familiar) and contributes to the social novelty preference exhibited by adult mice. In addition, the inventors find that the increase in ILA^{CRH} neuron density during the second postnatal week is responsible for a developmental shift in the social preference of young mice from familiar to novel conspecifics.

The object of the invention is the use of agonists that act on the corticotropin-releasing hormone in the prefrontal cortex and its receptor in the septum to treat individuals exhibiting social preference disorders such as separation anxiety disorder or exaggerated introversion.

The inventors identified a population of inhibitory neurons in the infralimbic region of the prefrontal cortex (ILA) that express the neuropeptide corticotropin-releasing hormone (CRH) and project to the rostro-dorsal region of lateral septum (rdLS). Release of CRH from ILA to rdLS during social interactions with familiar mice activates CRHR1-expressing neurons in rdLS, thereby suppressing interactions with familiar mice and contributing to social novelty preference.

Thus, the present invention relates to the use of CRH, a peptide that the inventors have demonstrated to be necessary to acquire an adult-like social preference based on its action through CRHR1, in patients with social anxiety disorders (SAD), social preference disorders or exaggerated introversion, as this will act directly on the central nervous system targeting CRHR1 within the brain.

In a first aspect, the invention relates a CRHR1 agonist for use in the treatment and/or prevention of social anxiety disorders.

The term "CRHR1" or "corticotropin releasing hormone receptor 1", as used herein, refers to a G-protein coupled receptor that binds neuropeptides of the corticotropin releasing hormone family that are major regulators of the hypothalamic-pituitary-adrenal pathway. The encoded protein is essential for the activation of signal transduction pathways that regulate diverse physiological processes including stress, reproduction, immune response and obesity.

The term "CRHR1 agonist", as used herein, refers to any agent that interacts with CRHR1 producing a biological response. Particularly, the CRHR1 agonist are molecules with full affinity (full agonists) based on its chemical binging properties: P_{Kd} ranging from 5.00 to 10.00. Being P_{Kd} value defined as the negative algorithm of the K_{d} (K_{d} defines the dissociation constant for a radiolabeled ligand determined by saturation binding analysis. It is the molar concentration of radioligand which, at equilibrium, occupies 50% of the receptors). According to these chemical standards, examples of current available CRHR1 full agonists include, without limitation to, corticotropin-releasing hormone (CRH) or corticotropin-releasing factor (CRF); preferably ovine, human, mouse or rat CRH or CRF; Urocortin 1, Sauvagine, Urocortin 2, Tyr0-CRF (human) and Stressin-1.

In a preferred embodiment of the present invention, the CRHR1 agonist is selected from the list consisting of: corticotropin-releasing hormone (CRH) or corticotropin-releasing factor (CRF), urocortin 1, sauvagine, urocortin 2, human Tyr0-CRF and Stressin-1.

In other preferred embodiment of the present invention, the CRHR1 agonist is the human corticotropin-releasing hormone (CRH) or corticotropin-releasing factor (CRF), preferably ovine CRH, human CRH, mouse CRH or rat CRH.

The term "CRH" or "corticotropin releasing hormone", also called corticotropin-releasing factor (CRF), refers to a 41 amino acid neuropeptide that plays a key role in the coordinated behavioral, autonomic, neuroendocrine and neuroimmune responses.

In other preferred embodiment of the present invention, the CRHR1 agonist is CRH, wherein said CRH comprises or consists of the aminoacid sequence: SEQ ID NO: 1. The sequences of human, mouse and rat CRH are identical, the SEQ ID NO: 1.
SEQ ID NO: 1
SEEPPISLDLTFH LLREVLEMARAEQLAQQAHSN RKLM EI

As described above, the present invention relates to the use of CRHR1 agonist for the treatment and/or prevention of social anxiety disorders, preferably separation anxiety disorder or avoidant personality disorders.

The term "social anxiety disorder", as used herein, refers to a common type of anxiety disorder which appears in social situations. A person with social anxiety disorder feels symptoms of anxiety or fear so intense in situations where they may be scrutinized, evaluated, or judged by others, such as speaking in public, meeting new people, dating, being on a job interview, answering a question in class, having to talk to a cashier in a store or everyday things, such as eating or drinking in front of others or using a public restroom For some people, this fear may get in the way of going to work, attending school, or doing everyday things. People with social anxiety disorder may worry about engaging in social situations for weeks before they happen. Social anxiety disorder usually starts during late childhood and may resemble extreme shyness or avoidance of situations or social interactions. It occurs more frequently in females than in males, and this gender difference is more pronounced in adolescents and young adults. Without treatment, social anxiety disorder can last for many years, or even a lifetime.

The signs and symptoms of social anxiety disorder include, without limitation to, blush, sweat, or tremble; have a rapid heart rate, feel their "mind going blank," or feel sick to their stomach; have a rigid body posture, or speak with an overly soft voice; find it difficult to make eye contact, be around people they don't know, or talk to people in social situations, even when they want to; feel self-consciousness or fear that people will judge them negatively; or avoid places where there are other people.

The term "separation anxiety disorder", as used herein, refers to a stage of anxiety for infants and toddlers, usually until the 3 years of age, which interferes with school or other daily activities or includes panic attacks or other problems. The signs and symptoms of separation anxiety disorder include, without limitation to, recurrent and excessive distress about anticipating or being away from home or loved ones; constant, excessive worry about losing a parent or other loved one to an illness or a disaster; constant worry that something bad will happen, such as being lost or kidnapped, causing separation from parents or other loved ones; refusing to be away from home because of fear of separation; not wanting to be home alone and without a parent or other loved one in the house; reluctance or refusing to sleep away from home without a parent or other loved one nearby; repeated nightmares about separation; frequent complaints of headaches, stomach aches or other symptoms when separation from a parent or other loved one is anticipated; or panic disorder and panic attacks.

The term "avoidant personality disorder", as used herein, refers to feelings of extreme social inhibition, inadequacy, and sensitivity to negative criticism and rejection. Avoidant personality disorder causes significant problems that affect the ability to interact with others and maintain relationships in day-to-day life. About 1% of the general population has avoidant personality disorder. The signs and symptoms of avoidant personality disorder include, without limitation to, avoiding work, social, or school activities for fear of criticism or rejection, low self-esteem, self-isolation, fear of saying the wrong thing, blushing, stammering, getting embarrassed or fear of rejection that often makes it difficult to connect with other people or maintain intimate relationships or close friendships.

According to the American Psychiatric Association's *Diagnostic and Statistical Manual of Mental Disorders* (DSM-5), a person diagnosed with avoidant personality disorder needs to show at least four of the following criteria:
- Avoids occupational activities that involve significant interpersonal contact, because of fears of criticism, disapproval, or rejection.
- Is unwilling to get involved with people unless they are certain of being liked.
- Shows restraint within intimate relationships because of the fear of being shamed or ridiculed.
- Is preoccupied with being criticized or rejected in social situations.
- Is inhibited in new interpersonal situations because of feelings of inadequacy.
- Views self as socially inept, personally unappealing, or inferior to others.
- Is unusually reluctant to take personal risks or to engage in any new activities because they may prove embarrassing.

A mental health professional can assess your symptoms, make an accurate diagnosis, and suggest the appropriate treatment options. Current treatments for patients suffering from Avoidant personality disorders are based on psychotherapy. Medication such as an antidepressant or anti-anxiety drug might be used to help manage the anxiety felt by people with this disorder. Although no medications are approved by the FDA to treat avoidant personality disorder. Nowadays there is a lack of efficient treatments against social anxiety disorders. Current available treatments are based mainly on behavioral therapy alone or combined with antidepressants such as selective serotonin reuptake inhibitors (SSRIs) or with general medication against anxiety (benzodiazepines). Even though these treatments might mild the symptoms, nowadays, there are no medications with an FDA-labeled indication for SAD.

As used herein, the term "prevention" means the avoidance of occurrence of the disease or pathological condition in a subject, particularly when the subject has predisposition for the pathological condition but has not yet been diagnosed. In the present invention, the disease or pathological condition is at least one social anxiety disorder, preferably separation or avoidant personality disorder.

As used herein, the term "to treat" or "treatment" refers to inhibiting the disease or pathological condition, i.e., stopping its development; relieving the disease or pathological condition, i.e., causing regression of the disease or pathological condition; and/or stabilizing the disease or pathological condition in a subject. In the present invention, the disease or pathological condition is at least a social anxiety disorder, preferably separation or avoidant personality disorder.

As understood by the person skilled in the art, the CRHR1 agonist, preferably the CRH, is comprised in a composition (hereinafter "the composition of the invention"), preferably a pharmaceutical composition.

The composition of the invention may be formulated for pharmaceutical administration, i.e., forming part of pharmaceutical products to be administered to the subject by any means of administration.

Additionally, the composition of the invention may comprise one or more components or compounds having any biological and/or pharmacological useful activity in the prevention and/or treatment of social anxiety disorders, preferably separation or avoidant personality disorders, in a subject.

In other preferred embodiment, the pharmaceutical composition comprises a pharmaceutically acceptable carrier and/or an excipient.

The term "excipient" refers to a substance that helps the absorption of any components or compounds of the composition of the invention, namely the CRHR1 agonist, or stabilizes the components or compounds and/or assists the preparation of the pharmaceutical composition in the sense of giving it consistency or flavors to make it more pleasant. Thus, the excipients may have the function, by way of example but not limited thereto, of binding the components (for example, starches, sugars or cellulose), sweetening, coloring, protecting the active ingredient (for example, to insulate it from air and/or moisture), filling a pill, capsule or any other presentation or a disintegrating function to facilitate dissolution of the components, without excluding other excipients not listed in this paragraph. Therefore, the term "excipient" is defined as that material that included in the galenic forms, is added to the active ingredients or their associations to enable their preparation and stability, modify their organoleptic properties or determine the physical and chemical properties of the pharmaceutical composition and its bioavailability. The "pharmaceutically acceptable" excipient must allow the activity of components or compounds of the pharmaceutical composition, that is, be compatible with the effect exerted by the CRHR1 agonist.

The "galenic form" or "pharmaceutic form" is the configuration to which the active ingredients and excipients are adapted to provide a pharmaceutical composition or a drug. It is defined by the combination of the form in which the pharmaceutical composition is presented by the manufacturer and the form in which it is administered.

The "vehicle" or "carrier" is preferably an inert substance. Carrier functions are to facilitate the incorporation of other components or compounds, allow better dosage and administration and/or give consistency and form to the pharmaceutical composition. Therefore, the carrier is a substance used in the drug to dilute any of the components or compounds of the pharmaceutical composition of the present invention to a given volume or weight; or that even without diluting these components or compounds, it is able to allow better dosage and administration and/or give consistency and form to the drug. When the presentation is liquid, the pharmaceutically acceptable carrier is the diluent. The carrier can be natural or unnatural. Examples of pharmaceutically acceptable carriers include, without being limited thereto, water, salt solutions, alcohol, vegetable oils, polyethylene glycols, gelatine, lactose, starch, amylose, magnesium stearate, talc, surfactants, silicic acid, viscous paraffin, perfume oil, monoglycerides and diglycerides of fatty acids, fatty acid esters petroetrals, hydroxymethylcellulose, polyvinylpyrrolidone and the like.

Furthermore, the excipient and the carrier must be pharmacologically acceptable, i.e., the excipient and the carrier are permitted and evaluated so as not to cause damage to the subject to whom it is administered.

In each case the presentation of the composition will be adapted to the type of administration used. Thus, the composition may be presented in the form of solutions or any other form of clinically permissible administration and in a therapeutically effective amount. The composition of the invention can be formulated into solid, semisolid or liquid preparations, such as tablets, capsules, powders, granules, solutions, suppositories, gels or microspheres.

Within the context of the present invention, a "therapeutically effective amount" is any amount of the component or compound of the pharmaceutical composition, which, when administered to a subject, is sufficient to produce the desired effect.

In other preferred embodiment of the invention, as knows the skilled person in the art, the therapeutically effective amount of CRHR1 agonist or the composition comprises CRHR1 agonist depend on the agonists selected for use.

In other preferred embodiment, the therapeutically effective amount of CRHR1 agonist or the composition comprises CRHR1 agonist, particularly of CRH, is a cumulative doses less than 300 µg/h, preferably less than 200 µg/h.

In other preferred embodiment, the therapeutically effective amount of CRHR1 agonist or the composition comprises CRHR1 agonist, particularly of CRH, for subcutaneous or intranasal application is between 1 and 30 µg/kg body weight.

The composition of the present invention may be administered by oral, parenteral (eg, intramuscular, intraperitoneal, intravenous, ICV, intracisternal injection or infusion, subcutaneous injection or implant, or intra-nasal administration) routes of administration, preferably formulated for intra-nasal administration. Thus, in other preferred embodiment of the invention, the CRHR1 agonist or the composition is formulated for intra-nasal administration.

### DESCRIPTION OF THE DRAWINGS

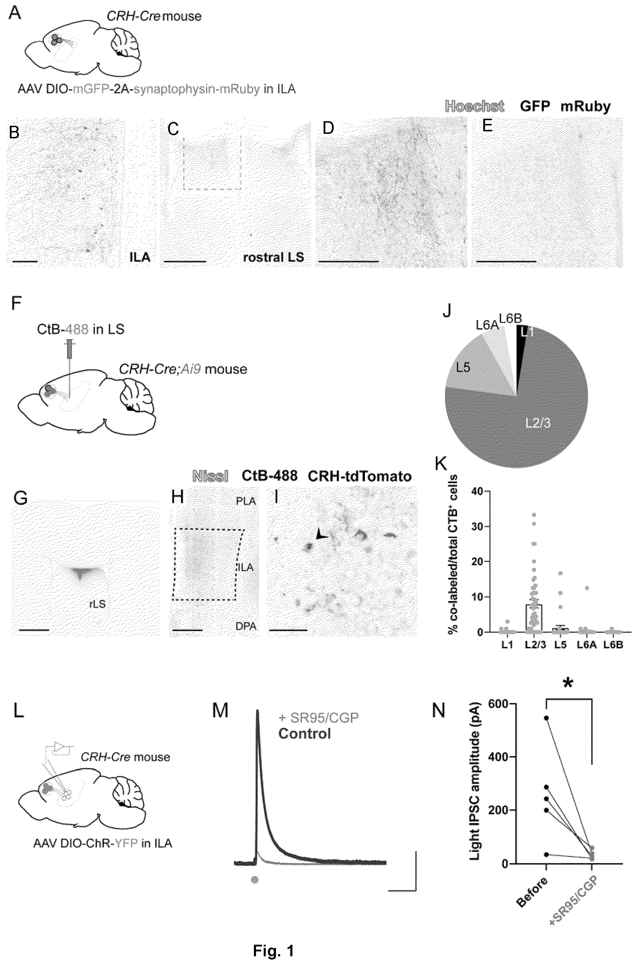

**Figure 1****. ILA^{CRH} cells project to rostro-dorsal LS.**
   **A.** CRH-Cre mice injected in ILA with AAV2/DJ hSyn.FLEX.mGFP.2A.Synatophysin-mRuby (AAV DIO-mGFP-2A-synaptophysin-mRuby). **B-E.** Immunohistochemistry images of an ILA (B) and LS (C-E) sections labeled for GFP (B-D) or mRuby (E) and Hoechst (B-E). Scale bars: 100 µm (B), 500 µm (C) and 200 µm (D-E). **F.** CRH-Cre;Ai9 mice injected in rdLS (LS) with CtB-488. **G.** Image of a coronal brain section containing the injection site. Scale bar: 1 mm. **H-I**. Images of coronal brain sections containing the mPFC. White arrowheads indicate CtB+ (CtB-488)/CRH+ (CRH-tdTomato) cells. Scale bar: 400 µm (H), 50 µm (I). **J.** Distribution of CtB+ cells in ILA (6 mice, L1, L2/3, L5, L6A, L6B). **K.** Percentage of co-labeled tdTomato+ and CtB+ over the total number of CtB+ cells per ILA layer. Each point from a different section. 4 sections / mice, N = 6 mice. Bar graph represents mean ± S.E.M. Nested one-way ANOVA, F4,25 = 24.20, p<0.0001. L. CRH-Cre mice injected in ILA with AAV2/9 EF1a.DIO.hChR2(E123T/T159C)-eYFP (AAV DIO-ChR-YFP in ILA). **M.** Electrophysiological traces from a rdLS neuron recorded in voltage-clamp configuration at +10 mV. Dot, stimulation of CRH+ fibers expressing Channelrhodopsin using 1 ms pulse of blue light to elicit an IPSC before and after application of SR95 and CGP (+SR95/CGP). Scale bars: 100 pA and 100 ms. **N**. IPSC amplitude (pA) before and after SR95 and CGP application (5 cells in 4 mice; paired t test, p=0.04).LS: Lateral septum, rdLS: rostro-dorsal Lateral septum, PLA: Prelimbic area of the prefrontal cortex, ILA: Infralimbic area of the prefrontal cortex, DPA: Dorsal peduncular area of the cortex, CtB: Cholera Toxin subunit B, L1: Layer 1 of the cortex, L2/3: Layer 2/3 of the cortex, L5: Layer 5 of the cortex, L6A: Layer 6A of the cortex, L6B: Layer 6B of the cortex, pA: picoAmps, ms: milliseconds, µm: micrometers, CRH: Corticotropin releasing hormone.

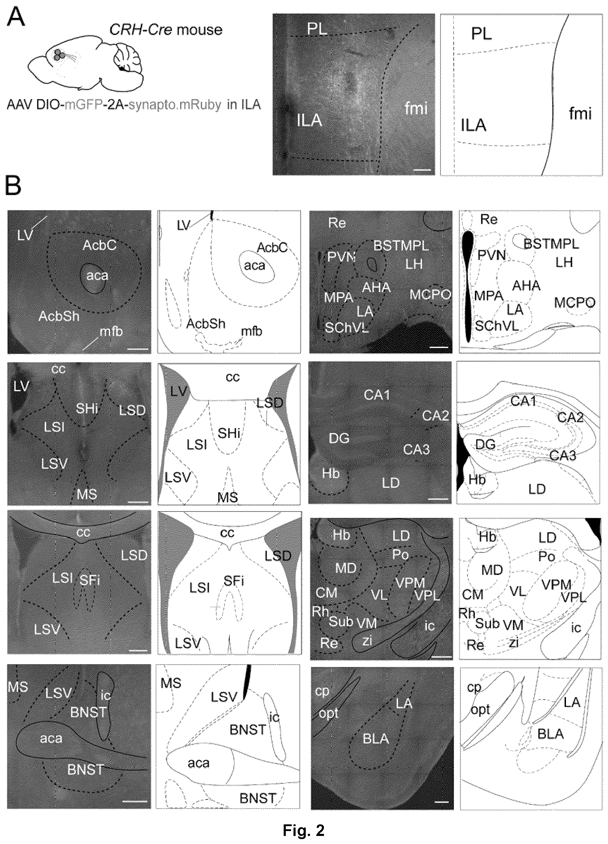

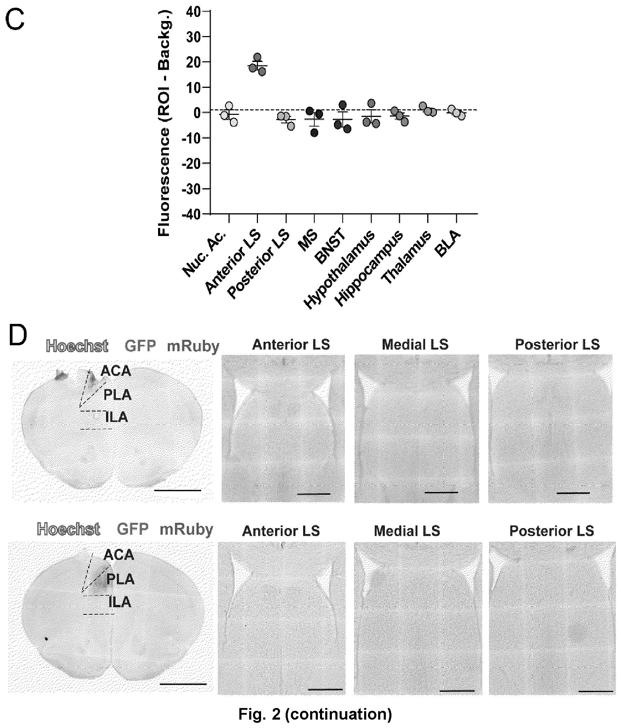

**Figure2****. ILA^{CRH} neurons send long-range projections to rLS exclusively.**
   **A.** CRH-Cre mice injected in ILA with AAV2/DJ hSyn.FLEX.mGFP.2A.Synatophysin-mRuby (AAV DIO-mGFP-2A-synapto.mRuby) and Immunohistochemistry image of the injection site. Scale bar: 200 µm. **B.** Immunohistochemistry images of several know target regions of the mPFC. Scale bars: 200 µm **C.** Quantification of the GFP signal in each region. Each dot is a different mouse. **D.** CRH-Cre mice injected in ACA or PLA with AAV2/DJ hSyn.FLEX.mGFP.2A.Synatophysin-mRuby. Immunohistochemistry images of mPFC and LS section showing injection site on the right and lack of projection in LS on the left. Scale bars: 2 mm (mPFC) and 500 µm (LS). PL: Prelimbic area, ILA: Infralimbic area, fmi: fimbria of corpus callosum, LV: Lateral ventricle, aca: anterior commissure, AcbC: nucleus accumbens core, mfb: medial forebrain bundle, Re: nucleus of reunions, PVN: paraventricular nucleus, MPA: medial preoptic area, BSTMPL: bed nuclei of the stria terminalis, LH: lateral hypothalamic area, AHA: anterior hypothalamic area, LA: lateral amygdalar nucleus, MCPO: magnocellular preoptic nucleus, SChVL: suprachiasmatic nucleus ventrolateral, cc: corpus callosum, Shi: septohippocampal nucleus, LSI: lateral septum intermediate part, LSV: lateral septum ventral part, LSD: lateral septum dorsal part, MS: medial septum, CA1: field of CA1 of the hippocampus, CA2: field of CA2 of the hippocampus, CA3: field of CA3 of the hippocampus, DG: Dentate gyrus, Hb: Habenula, LD: laterodorsal nucleus of the thalamus, SFi: septofimbrial nucleus, MD: mediodorsal nucleus of the thalamus, Po: posterior nucleus of the thalamus, VPM: ventromedial nucleus of the thalamus, VL: ventrolateral nucleus of the thalamus, VPL: ventroposterior lateral nucleus of the thalamus, Rh: Rhomboid nucleus, zi: zona incerta, ic: internal capsule, Sub: submedius thalamic nucleus, BNST: bed nuclei of the stria terminalis, cp: caudate putamen, opt: optic tract, BLA: basolateral amygdala, LA: lateral amygdala, mPFC: medial prefrontal cortex.

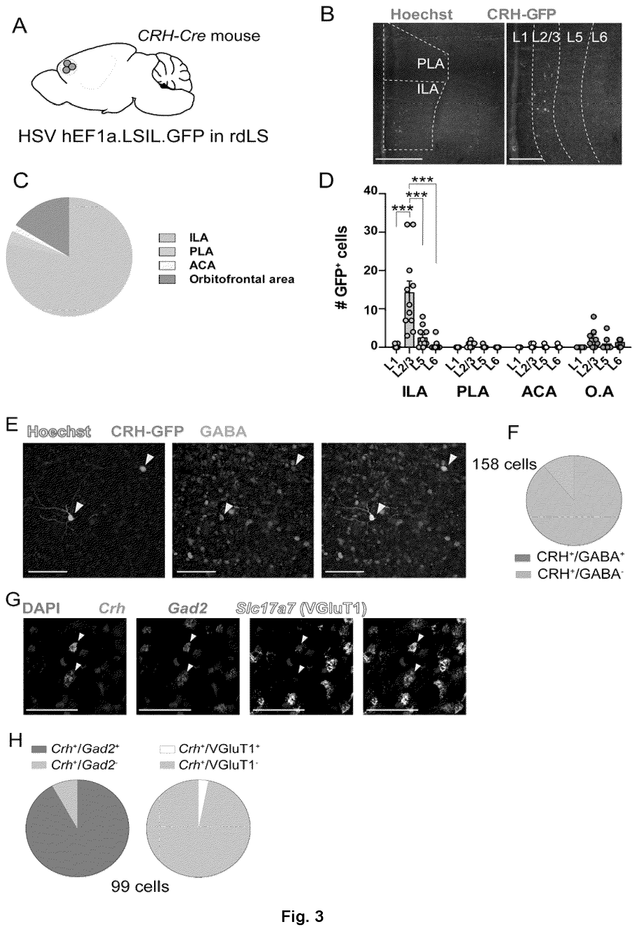

**Figure 3****. Molecular identity of ILACRH cells.**
   **A.** CRH-Cre mice injected with HSV hEF1a.LSIL.GFP in rdLS. 3 mice. **B.** Images of the mPFC and ILA showing CRH/GFP⁺ cells retrogradely labeled and Hoechst. Scale bars: 500 µm (left) and 200 µm (right) (mice L1, L2/3, L5, L6). **C.** Distribution of GFP+ cells per regions (ILA, PLA, ACA, Orbitofrontal area). **D.** Number of GFP+ cells per region (ILA, PLA, ACA, Orbitofrontal area O.A). and layers. (C-D) 3 sections/mice. N = 3 mice. **E.** Immunohistochemistry images of the ILA labeled for GABA (Hoechst, CRH-GFP and GABA). Scale bars: 100 µm. **F.** Associated distribution of GFP⁺ cells (CRH+/GABA+ or CRH+/GABA-). N = 3 mice. **G.** In situ hybridization images of the ILA labeled for *Crh, Gad2* and *Slc17a7* (VG!uT1). N = 3 mice. Scale bars: 50 µm. H. Associated distribution of *Crh⁺* cells (*Crh+*/*Gad2+, Crh+*/*Gad2-, Crh+*/*VgluT1+, Crh+*/*VgluT1-).* L1: Layer 1 of the prefrontal cortex, L2/3: Layer 2/3 of the prefrontal cortex, L5: Layer 5 of the prefrontal cortex, L6: Layer 6 of the prefrontal cortex. ILA: Infralimbic area, PLA: Prelimbic area, ACA: Anterior cingulate area, OA: orbitofrontal area.

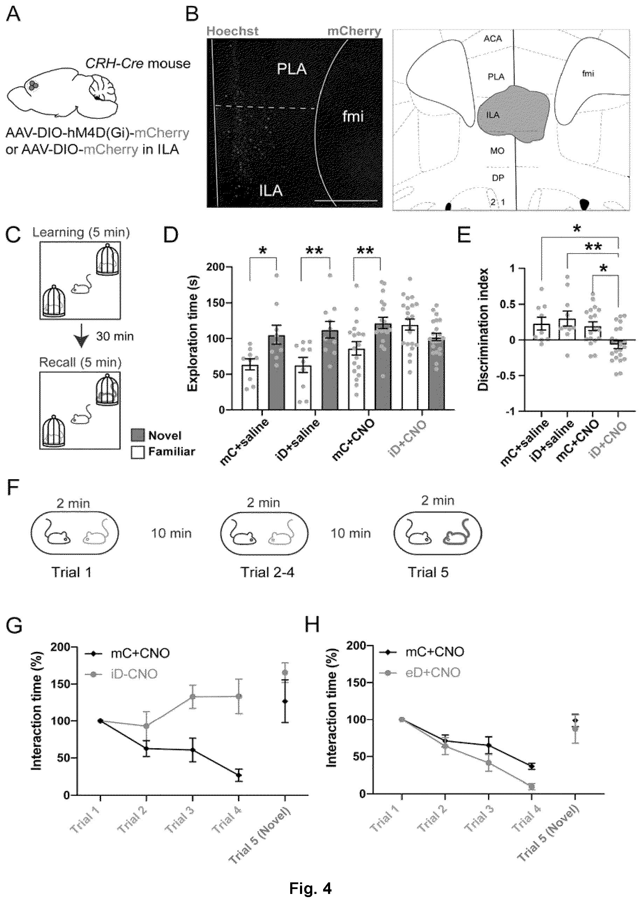

**Figure 4****. ILA^{cRH} cells support social novelty preference and familiarization.**
   **A.** CRH-Cre mice injected in ILA with AAV2/8 hSyn.DIO.hM4D(Gi)-mCherry (AAV-DIO-hM4D(Gi)-mCherry) (iDREADD) or AAV2/8 hSyn.DIO.mCherry (AAV-DIO-mCherry). **B.** Left: immunohistochemistry image showing the extent of iDREADD expression in several sections (PLA, ILA, fmi, ACA, MO, DP). Scale bar: 500 µm. Right: maximal extent of iDREADD expression across several mPFC sections. **C.** Schematic of the social novelty preference test. **D.** Interaction time with novel (dark grey) or familiar (light grey) mouse during recall in mice expressing mCherry (mC) or hM4Di (iD). Both groups were injected with saline (left groups) or 5 mg/kg of the DREADD agonist CNO (right groups). Grey dots are different mice. 3-way ANOVA F (novelty x injection x virus)1,108 = 3.471, p = 0.02. Sidak's multiple comparison tests novel vs. familiar: mC + saline, p = 0.04; iD + saline, p = 0.006; mC + CNO, p = 0.008; iD + CNO, p = 0.3. **E.** Discrimination indexes for social novelty preference of the four groups during recall trial. One-sample t tests compared to 0: mC + saline, p = 0.04; iD + saline, p = 0.02; mC + CNO, p = 0.006; iD + CNO, p = 0.2. 2-way ANOVA: F(virus x injection)1, 54 = 4.7, p = 0.03; F(virus )1, 54 = 7.1, p = 0.01; F(injection)1, 54 = 1.5, p = 0.2. Tukey's multiple comparison tests compared to the iD + CNO group: mC + saline, p = 0.04; iD + saline, p = 0.004; mC + CNO, p = 0.02. **F.** Schematic of the repetitive social presentation test. **G.** Normalized interaction times during social presentations (inhibitory DREADD-expressing mice (iD) and controls (mC) injected with CNO). 8 mice per group. Two-way ANOVA, F(trial1-4 x virus)3,55 = 5.44, p = 0.002; F(trial)3,55 = 1.28, p = 0.2; F(virus)3,55 = 26.82, p < 0.0001. Post-hoc Sidak's multiple comparison tests between mC and iD groups, trial 2 p = 0.4, trial 3 p = 0.004 and trial 4 p < 0.0001. **H.** Normalized interaction times during repetitive social presentation test in CRH-Cre mice injected in ILA with AAV5 hSyn.DIO.hM3D(Gq)-mCherry (excitatory DREADD (eD)) or with AAV5 hSyn.DIO.mCherry as a control (mC). 8 mice per group. Two-way ANOVA: F(trial1-4 x virus)3,56 = 1.36, p = 0.26; F(trial)3,56 = 33.05, p < 0.0001; F(virus)3,56 = 6.765, p = 0.012. PLA: Prelimbic area, ILA: Infralimbic area, ACA: Anterior cingulate area, MO: Motor area, DP: Dorsal peduncular area, fmi: fimbria of the corpus callosum.

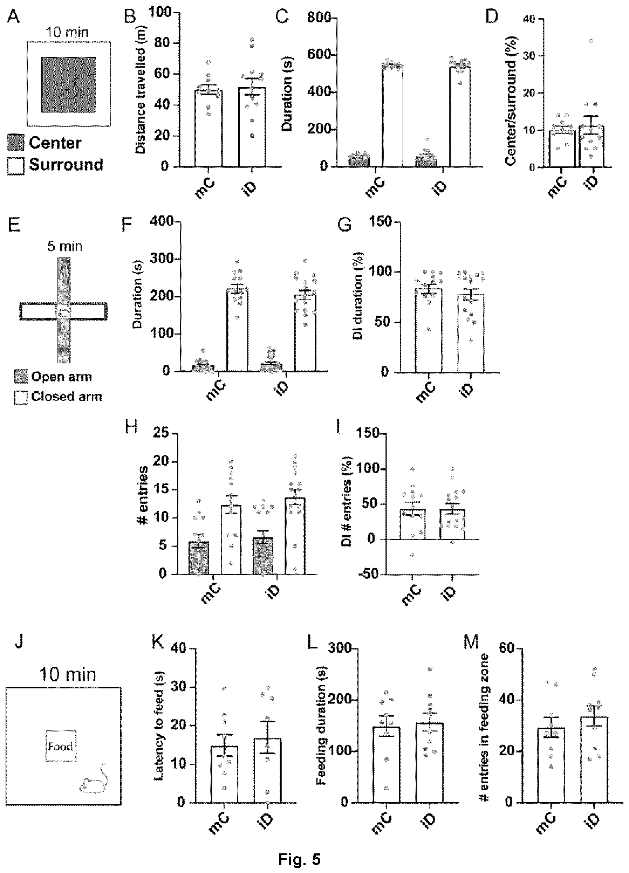

**Figure 5****. Locomotion, anxiety and feeding behavior are not affected by chemogenetic silencing of ILACH neurons.**
   CRH-Cre mice injected in ILA with AAV2/8 hSyn.DIO.hM4D(Gi)-mCherry (iD) or AAV2/8 hSyn.OIO.mCherry (mC). **A.** Schematic of the open-field test. Controls (mC) inhibitory DREADD (iD). **B.** Total distance travelled during open-field test. Controls (mC) inhibitory DREADD (iD) **C.** Time spent in the center or surround of the open-field. Controls (mC) inhibitory DREADD (iD) **D.** Ratio of the time spent in the center/surround. Controls (mC) inhibitory DREADD (iD) **E.** Schematic of the elevated-plus maze test. **F.** Time spent in the open or closed arms. Controls (mC) inhibitory DREADD (iD) **G.** Discrimination indexes for closed arm preference using the time spent in the arms. Controls (mC) inhibitory DREADD (iD) **H.** Number (#) of entries in the open or closed arms. Controls (mC) inhibitory DREADD (iD) **I.** Discrimination indexes for closed arm preference using the number of arm entries. Controls (mC) inhibitory DREADD (iD) **J.** Schematic of the novelty suppressed feeding test. **K.** Latency to feed. Controls (mC) inhibitory DREADD (iD) **L.** Feeding duration. Controls (mC) inhibitory DREADD (iD) **M.** Number (#) of entries in the feeding zone. Controls (mC) inhibitory DREADD (iD) For the entire figure, bar graphs represent mean ± S.E.M. Grey dots are different mice.

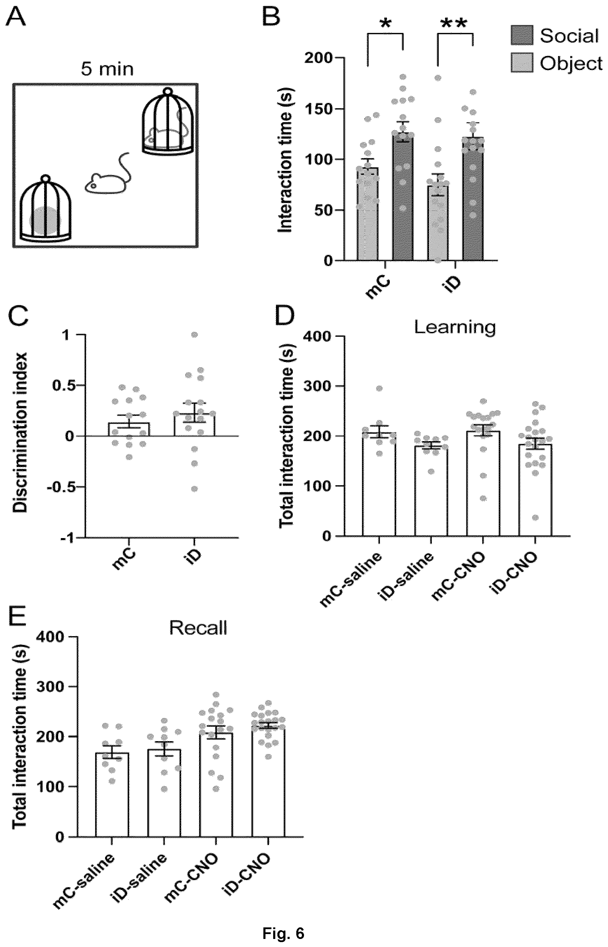

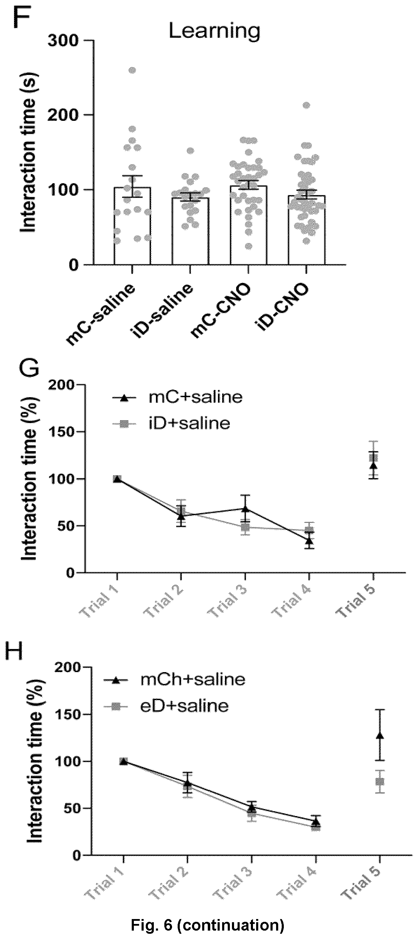

**Figure 6****. Social behavior controls for chemogenetic silencing of ILACRH cells.** CRH-Cre mice injected in ILA with AAV2/8 hSyn.DIO.hM4D(Gi)-mCherry (iD) or AAV2/8 hSyn.DIO.mCherry (mC). **A.** Schematic of the sociability test. **B.** Interaction times with mouse (social) (dark grey) or object (light grey). Paired t tests: p = 0.01, p = 0.009. **C.** Discrimination indexes for social preference. Controls (mC) inhibitory DREADD (iD) One-sample t tests compared to 0: p = 0.03 and p = 0.02. Unpaired t test: p = 0.4. **D-E.** Total interaction times during learning (D) or recall trial of the social novelty preference test (E). Two-way ANOVAs F(CNO vs. saline)1,55 = 0.19, p = 0.7 and F(CNO vs. saline)1,55 = 14.51, p = 0.0004 respectively. **F.** Interaction time with each novel mouse during the learning trial of the social novelty preference test. One-way ANOVA, F(CNO vs. saline)1,114 = 0.1109, p = 0.7. **G.** Normalized interaction times during social presentations (inhibitory DREADD-expressing mice and controls injected with saline). 8 mice per group. Two-way ANOVA: F(trial1-4 x virus)3,56 = 1.03, p = 0.39; F(trial)3,56 = 14.72, p < 0.0001; F(virus)1,56 = 0.003, p = 0.9. **H.** Normalized interaction times during social presentations (excitatory DREADD-expressing mice and controls injected with saline) (excitatory DREADD: eD). 8 mice per group. Two-way ANOVA: F(trial1-4 x virus)3,56 = 0.09, p = 0.96; F(trial)3,56 = 33.86, p < 0.0001; F(virus)1,56 = 0.72, p = 0.4. For the entire figure, bar graphs represent mean ± S.E.M. Grey dots are from different mice except for (F) where two observations per mice were made since the mice interacted with two novel mice during the learning trial.

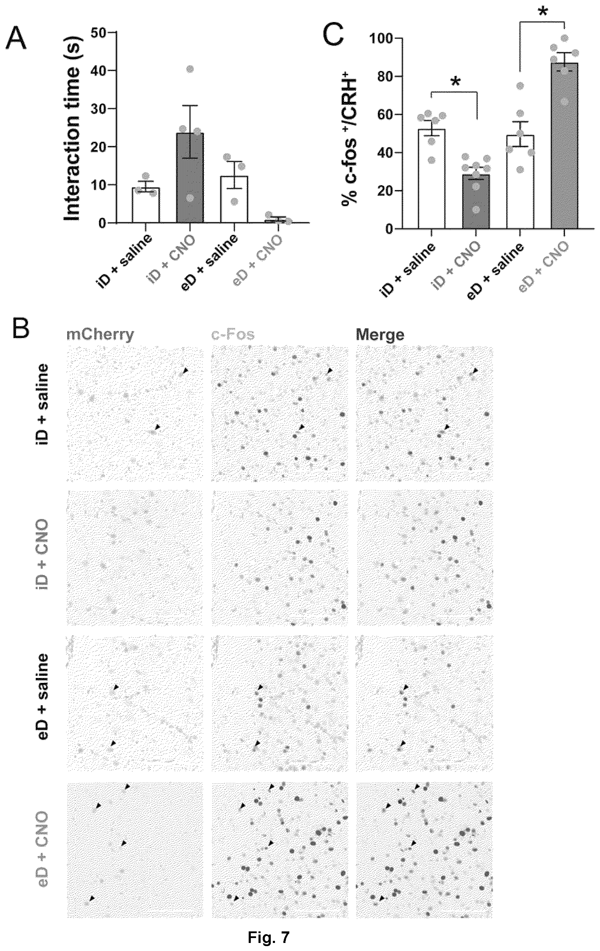

**Figure 7****. DREADDs modulate ILACRH cells activity.**
   CRH-Cre mice injected in ILA with AAV2/8 hSyn.DIO.hM4D(Gi)-mCherry (iD) orAAV5 hSyn.DIO.hM3D(Gq)-mCherry (excitatory DREADD eD). Mice received CNO or saline i.p. injection 30 min before being presented to a familiar animal for 2 min. Mice were thereafter perfused and processed for c-fos labelling. **A.** Interaction times. Each point is one mouse. Inhibitory DREADD: iD, control virus: mC, excitatory DREADD: eD. **B.** Immunohistochemistry images of the Infralimbic region (ILA) labelled for c-fos (dark grey) or mCherry (light- grey). Scale bars: 100 µm. **C.** Percentage of mCherry+ cells in ILA expressing c-fos. Minimum of 3 mice per group. 2 observations per mice. Nested t tests: p = 0.01 and p = 0.02.

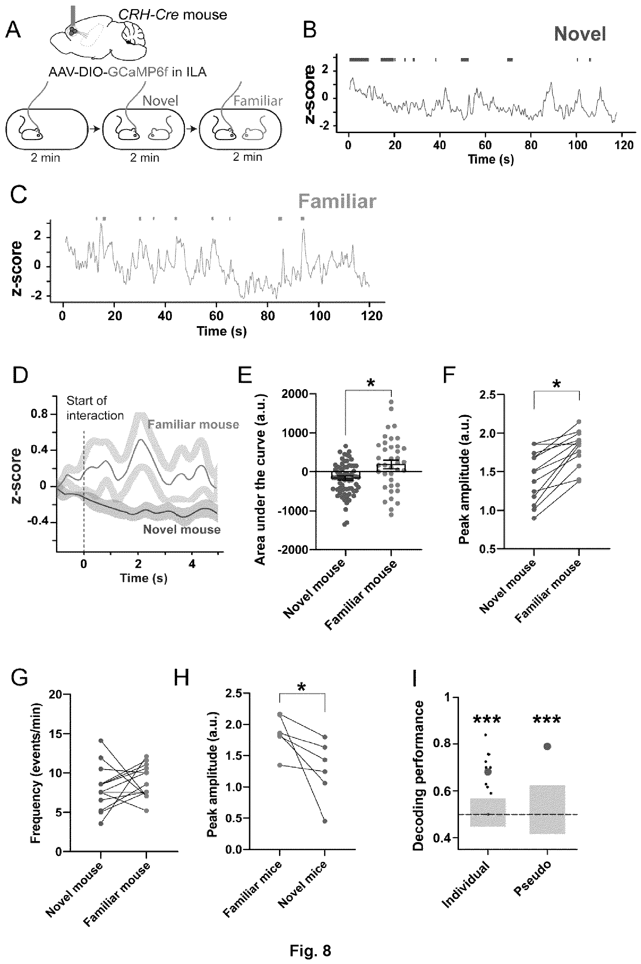

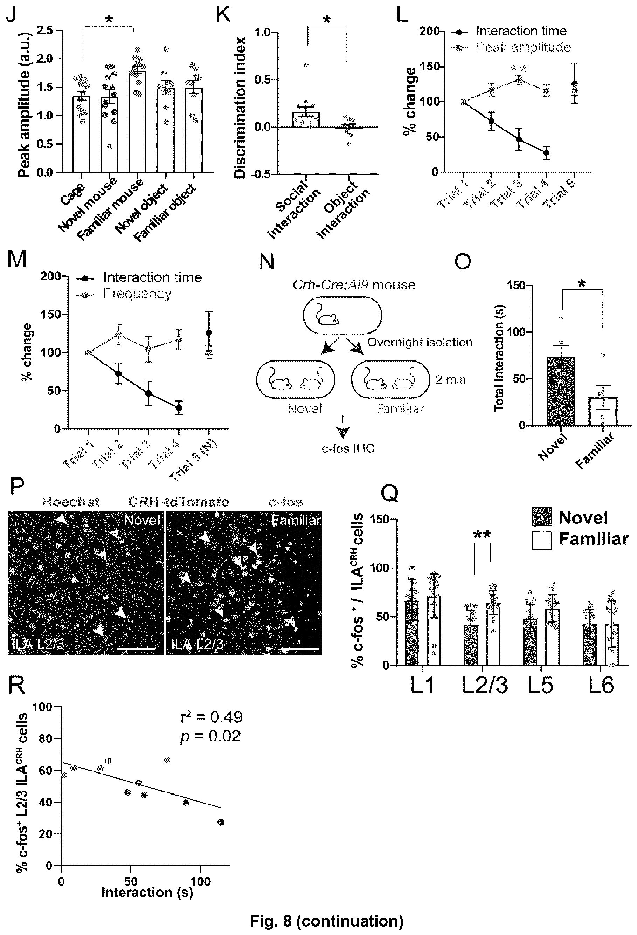

**Figure 8****. ILACRH cells respond preferentially to familiar mouse presentation.**
   **A.** CRH-Cre mice injected in ILA with AAV2/1 syn.FLEX.GCaMP6f (AAV-DIO-GCaMP6f) and implanted with an optical ferrule over Infralimbic region (ILA) (top). Schematic of the fiber-photometry recording experiment (bottom). Novel mouse (dark grey) and familiar mouse (light grey). **B-C.** Example traces of recording during presentation of a novel (B) or familiar (C) mouse to the same test mouse. Interaction bouts intervals are shown above each trace. The z-score represents the magnitude of the neuronal response. Time in seconds (s). **D.** Peri-stimulus time histogram during social interaction with novel or familiar mouse, 5 mice. **E.** Area under the curve during familiar and novel mouse interaction. Each point is an interaction, 5 mice. One-sample nested t tests: Familiar response vs. 0, p = 0.003, Novel responses vs. 0, p = 0.005. Nested t test between groups: p = 0.01. a.u.: arbitrary units **F.** Average peak amplitude of the z-score during social presentations of a novel then familiar mouse. For panels F, G and J, each dot is a different recording session using 5 mice. Nested t test between groups: p = 0.02. **G.** Frequency (events/minute) of calcium events during presentation of a novel then familiar mouse. Nested t test: p = 0.4. **H.** Average peak amplitude of the z-score during inverted presentation of a familiar then a novel mouse. 3 mice per groups. 2 observations per mice. Nested t test, p = 0.03. **I.** Decoding performance for familiarity versus novelty from individual recordings or pseudo-simultaneous data. Small black dots on the left are the results from individual recording sessions (N = 5 mice), using 20 cross-validation iterations, large black dot is the average. Large dark dot on the right is the result of pseudo-population analysis from 100 cross-validation iterations. Grey areas denote chance level computed using permutation tests (2.5 - 97.5 percentiles in distribution of shuffled decoding performances). In both cases, statistical significance is determined by the probability of drawing the observed decoding performance from the distribution of shuffled decoding performances (null-hypothesis). p < 0.001 (two-tailed permutation test, see Methods). **J.** Average peak amplitude during each type of presentation (novel then familiar experiments only). Nested One-way ANOVA F4,16 = 24.20 followed by Tukey's multiple comparison test: cage vs. novel mouse p = 0.9; cage vs. familiar mouse p = 0.03; cage vs. novel object p = 0.7; cage vs. familiar object p = 0.7. **K.** Discrimination indexes for familiarity preference calculated from z-scores during mouse or object presentation. Each point is one recording session. N = 5 mice. One-sample t tests compared to 0: p = 0.001 and p = 0.3 respectively. Unpaired t test between groups taking the average value per mice: p = 0.03. **L.** Fiber-photometry recording during repetitive social presentation test (10 sessions in 5 mice). One-sample t tests compared to: trial 1 p = 0.06; trial 3 p = 0.002 and trial 4 p = 0.04. **M.** Frequency of calcium events during repetitive social presentation test (10 sessions in 5 mice). **N.** CRH-Cre;Ai9 mice were presented with novel or familiar mice after overnight isolation before being processed for immunohistochemistry. **O.** Interaction times following 2 min social presentation. Unpaired t test novel vs. familiar interaction time, p = 0.04. **P.** Immunohistochemistry images of c-fos labelling in Infralimbic area (ILA) layer 2/3. Arrowheads on the left show that the majority of cells are: c-fos- / tdTomato+ cells. Arrowheads on the right show that the majority of cells are: c-fos+ / tdTomato+ cells. Scale bars: 100 µm. **Q.** Percentage of ILA^{CRH} cells positive for c-fos per layer. Each point corresponds to each side of 2 sections. 5 mice per group. Nested t test, p = 0.003. **R.** Percentage of layer 2/3 ILA^{CRH} cells positive for c-fos vs. interaction time during social interaction with novel (dark grey) or familiar (light grey) mouse. Each point represents one mouse. For the entire figure, bar graphs represent mean ± S.E.M.

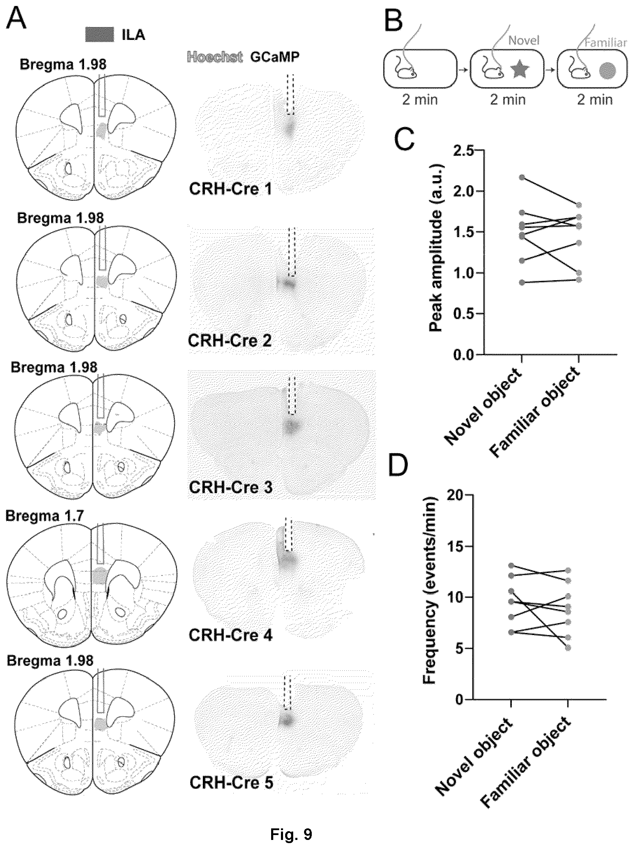

**Figure 9****. Fiber-photometry recordings of ILA^{CRH} cells.**
   **A.** Immunohistochemistry images of GCaMP expression and lens implant in the ILA of recorded *CRH-Cre* mice. Scale bars: 2 mm. On the right: sections with Hoechst (light grey) and GCaMP (dark grey) **B.** Schematic of the object presentation experiment. **C.** Average peak amplitude of the z-score during presentation of a novel or familiar object. Dots are different sessions using 3 mice. Nested t test between groups, *p*=0.9. **D.** Frequency of calcium events during presentation of a novel or familiar object. Dots are different sessions using 3 mice. Nested t test between groups, *p*=0.4.

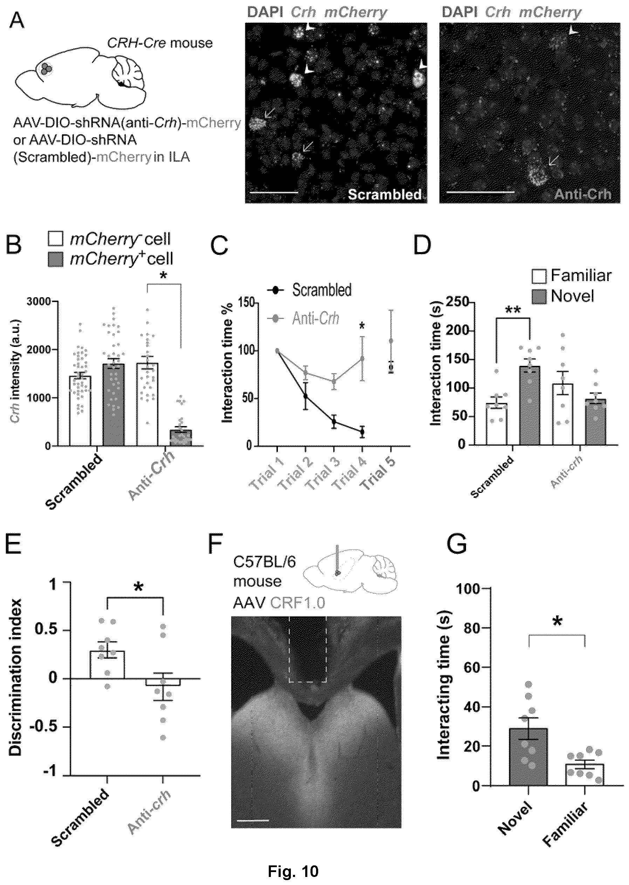

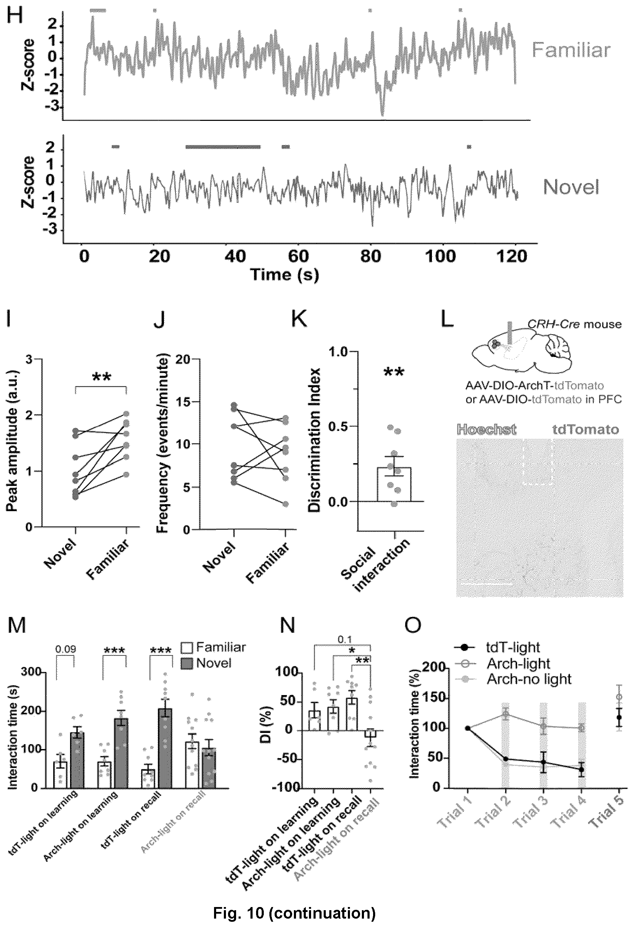

**Figure 10****. CRH release from ILA in rdLS suppresses social interactions with familiar mice and supports social novelty preference.**
   **A.** *CRH-Cre* mice injected in ILA with AAV2/9 CMV-DIO-(mCherry-U6)-shRNA(anti-*Crh*) (AAV-DIO-shRNA(anti-Crh)-mCherry) to downregulate *Crh* or control AAV2/9 CMV-DIO-(mCherry-U6)-shRNA(scrambled) (AAV-DIO-shRNA(Scrambled)-mCherry)) (right). In situ hybridization images of ILA slices expressing the scrambled shRNA (middle) or the shRNA against *Crh* (right) labelled for *mCherry* and *Crh.* White arrows denote CRH⁺ neurons that do not express the virus. White arrowheads on the left panel denotes CRH⁺ cells expressing the anti-*Crh* shRNA, with reduced level of *Crh.* White arrowheads on the right panel denote CRH⁺ neurons expressing the scrambled shRNA, with intact *Crh* level. Scale bars: 50 µm. B. Quantification of *Crh* expression. In each slice neurons were classified as to whether they were uninfected or infected with virus based on *mCherry* expression (3 mice per group; each point is a different neuron). Nested one-way ANOVA F_{3,8} = 6.41, *p* = 0.016 followed by Tukey's multiple comparison test, anti-*Crh* + mCherry⁺ vs. anti-*Crh* + mCherry⁻, *p* = 0.03. **C.** Normalized interaction time during the repetitive social presentation test in mice expressing scrambled or anti-*Crh* shRNAs. 4 mice per group. Two-way ANOVA; F(trial₁₋₄ x virus)_{3,24} = 4.4 p = 0.01; F(trial)_{3,24} = 9.6, *p* = 0.0002; F(virus)_{3,24} = 21.9, *p* < 0.0001 followed by Tukey's multiple comparison test between scrambled and anti-Crh groups: trial 2, *p* = 0.8; trial 3, *p* = 0.2; trial 4, *p* = 0.009. D. Interaction time with familiar (white) or novel (dark grey) mouse during the recall trial of the social novelty preference test in mice expressing scrambled or anti-*Crh* shRNAs. Grey dots are different mice. 2-way ANOVA F(novelty x virus)_{1,28} = 11.53, *p* = 0.002. Sidak's multiple comparison tests novel vs. familiar: scrambled, *p =* 0.004; anti-Crh, *p =* 0.3. Paired t test: *p* = 0.0095, *p* = 0.6. **E.** Discrimination indexes for social novelty preference during recall trial. Grey dots are different mice. Unpaired t test: p = 0.03. **F.** Top: C57BL/6J wild-type mice injected in rdLS with AAV2/9 Syn.CRF1.0 and implanted with an optical ferrule above rdLS. Bottom: immunohistochemistry image showing CRF1.0 expression in rdLS and the optical ferrule implanted above the injection site. Scale bar: 300 µm. **G.** Bar graph showing the interaction time with novel and familiar mice. 8 mice. Paired *t* test, *p* = 0.002. **H.** Trace of a representative fiberphotometry recording during interaction with a familiar mouse or a novel mouse. Interaction bouts are shown above each trace. **I.** Average peak amplitude of the z-score during presentation of a novel or familiar mouse. 8 mice. Paired *t* test: *p* = 0.008. **J.** Frequency of events during presentation of a novel or a familiar mouse. 8 mice. Paired *t* test: *p* = 0.5. K. Discrimination index for social familiarity preference calculated from z-scores. 8 mice. One-sample *t* tests compared to 0: *p* = 0.008. **L.** (top) *CRH-Cre* mice injected in ILA with AAV2/2 CAG.FLEX.ArchT-tdTomato or control AAV2/2 CAG.FLEX.tdTomato. (bottom) Optical ferrule implant is above rdLS. Scale bar: 500 µm. Light grey (Hoechst) and black (tdTomato). **M.** Interaction time with familiar (white) or novel (dark grey) mouse during the recall trial of the social novelty preference test in the same mice. Laser was on during the learning or recall trial. Each dot is a mouse. 3-way ANOVA F(novelty x light x virus)_{1,62}=14,44, p=0.007. Šidák's multiple comparison tests novel vs. familiar: *p*=0.03, 0.004, <0.0001 and 0.8. N. Discrimination index for social novelty preference during recall trial of the social novelty preference test. One-sample t tests: *p*=0.04, 0.007, 0.0007 and 0.4. Two-way ANOVA: F(virus x light)_{1,31}=6.232, *p*=0.01. F(light)_{1,31}=1.578, *p*=0.2; F(virus)_{1,31}=5.701, *p*=0.02. Šidák's multiple comparison tests: *p*=0.09, 0.04 and 0.003. O. Normalized interaction time during the repetitive social presentation test in the same mice. The laser was on during trials 1 to 4 of the Arch-light and mC-light groups (4 mice and 3 mice respectively). Laser was not on for the Arch-no light group (4 mice). Two-way ANOVA: F(trial₁₋₄ x virus)_{6,32} = 5.84, *p* = 0.0003; F(trial)_{3,32} = 14.35, *p* < 0.0001; F(group)_{2,32} = 49.32, *p* < 0.0001. For the entire figure, bar graphs represent mean ± S.E.M. Grey dots are different mice.

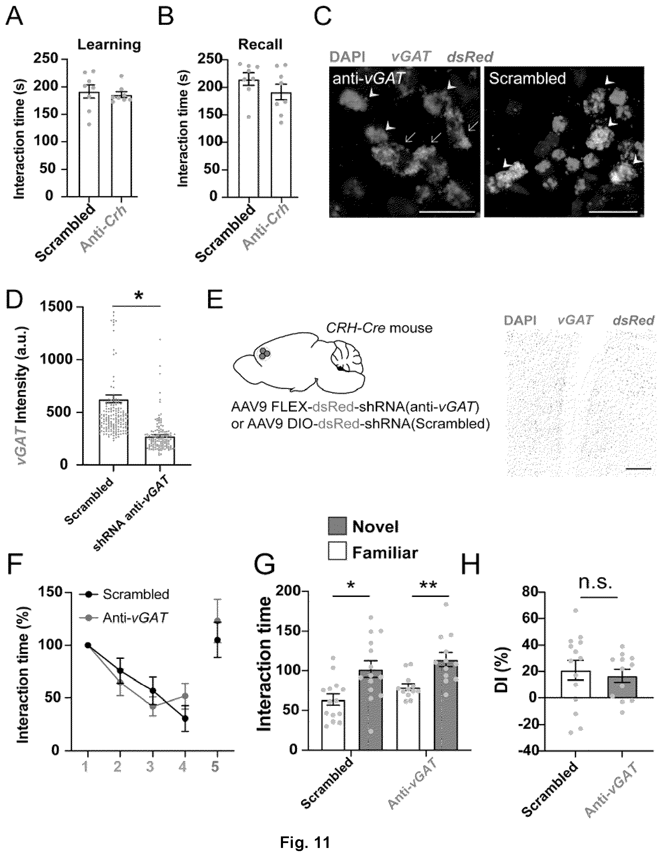

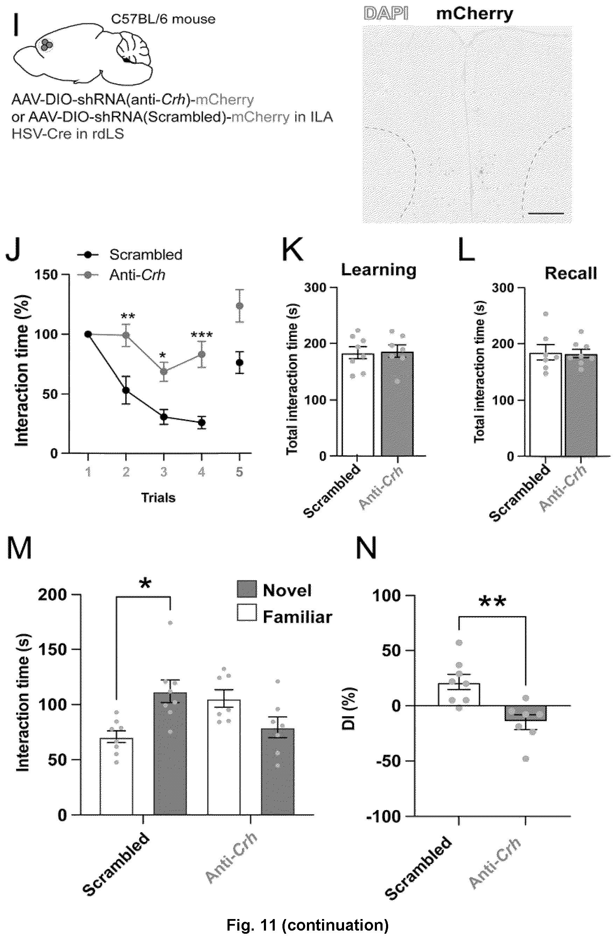

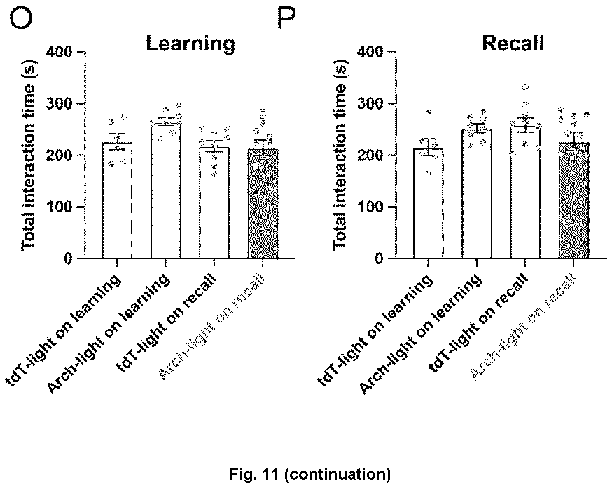

**Figure 11****. Social behavior controls for CRH release from ILA to rdLS. A-B.** Total interaction time during the learning (A) and recall (B) phases of the social novelty preference test following *Crh* knock-down in ILA^{CRH} neurons (Scrambled and Anti-Crh). C. In situ hybridization against DAPI, *vGAT* and *dsRed* in the Infralimbic area (ILA) of *CRH-Cre* mice injected with AAV2/9 hSyn.FLEX.dsRed-shRNA(anti-*vGAT*) or control AAV2/9 hSyn.FLEX.dsRed-shRNA(scrambled). Cells that express the shRNA against *vGAT* (Left white arrowheads) express *dsRed* but no more *vGAT* unlike nearby *vGAT*⁺ cells that did not incorporate the virus (white arrows). Cells expressing the scrambled shRNA express *dsRed* and retain *vGAT* expression (right white arrowheads). Scale bars 50 µm. **D.** Quantification of *vGAT* intensity in *dsRed*⁺ neurons in mice injected with each virus (Scrambled or shRNA anti-vGAT). Each point is a cell. 4 and 3 mice per group. Nested *t* test: *p* = 0.02. **E.** *CRH-Cre* mice injected in ILA with AAV2/9 hSyn.FLEX.dsRed-shRNA(anti-*vGAT*) or control AAV2/9 hSyn.FLEX.dsRed-shRNA(scrambled) (left). In situ hybridization picture against *vGAT* and *dsRed* showing viral expression (right). Scale bar 500 µm. **F.** Normalized interaction times during repetitive social presentation following expression of a shRNA against *vGAT (Anti-vGAT)* or a scrambled shRNA in ILA^{CRH} cells. Two-way ANOVA; F(trial₁₋₄ x virus)_{3,60} = 1.204, *p* = 0.3; F(trial)_{3,60} = 12.77, *p* < 0.0001; F(virus)_{1,60} = 0.031, *p* < 0.9. **G.** Interaction time during recall trial of the SNP test following expression of a shRNA against *vGAT* or a scrambled shRNA in ILA^{CRH} cells. Paired *t* tests *p* = 0.01, *p* = 0.008. **H.** Discrimination index during time during recall trial of the SNP test. One sample t test vs. 0: *p* = 0.01 and p = 0.006. Unpaired *t* test, *p* = 0.7. **I.** C57BU6 mice injected with HSV hEF1a.Cre in rdLS and AAV2/9 CMV-DIO-(mCherry-U6)-shRNA(anti-*Crh*) or AAV2/9 CMV-DIO-(mCherry-U6)-shRNA(scrambled) in ILA (left). Immunohistochemistry picture of ILA (right). Scale bar 500 µm. Dapi in light grey and mCherry in drak grey. **J.** Normalized interaction times during repetitive social presentation following expression of a shRNA against *Crh* (Anti-Crh) or a scrambled shRNA in retrogradely targeted ILA cells. Two-way ANOVA; F(trial₁₋₄ x virus)_{3,32} = 5.19, *p* = 0.005; F(trial)_{3,32} = 17.98, *p* < 0.0001; F(virus)_{1,32} = 42.01, *p* < 0.0001 followed by Tukey's multiple comparison test comparing anti-Crh and scrambled expressing mice for each trial (trial 2, *p* = 0.004; trial 3, *p* = 0.03, trial 4, *p* = 0.0002). K-L. Total interaction time during learning (K) and recall (L) trials of the SNP test. M. Interaction time during recall trial of the SNP test. Paired *t* tests *p* = 0.03, *p* = 0.06. N. Discrimination index during time during recall trial of the SNP test. One sample t test vs. 0: *p* = 0.02 and *p* = 0.07. Unpaired t test, *p* = 0.002. **O-P.** Total interaction time during learning (O) and recall (P) of the social novelty preference test following Arch or mCherry expression in ILA^{CRH} and silencing of their terminals in LS. For the entire figure, bar graphs represent mean ± S.E.M. Grey dots are different mice.

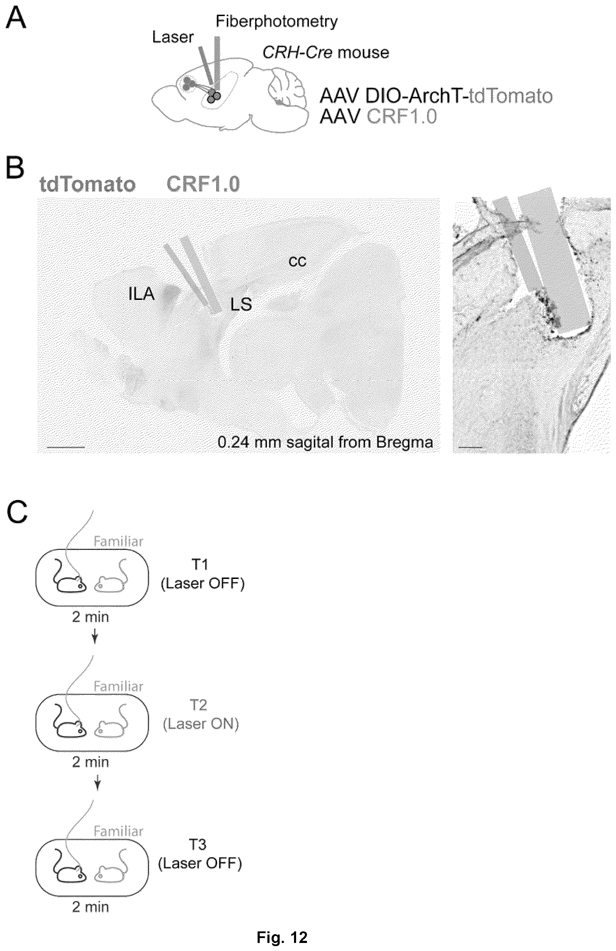

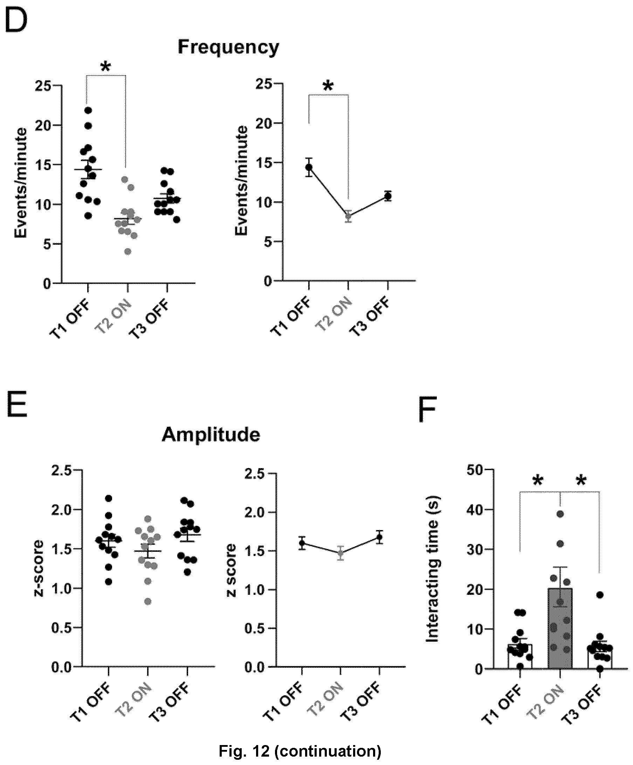

**Figure 12****. Arch-mediated silencing of ILA^{CRH} neuron terminals in rdLS reduces CRH release.**
   **A.** *CRH-Cre* mice injected with AAV2/2 CAG.FLEX.ArchT-tdTomato in ILA and AAV2/9 syn.CRF1.0 in rdLS and implanted with two optical ferrules above rdLS. **B.** Immunohistochemistry picture showing fiber tracts and viral expression. Scale bars: 1 mm and 200 µm. **C.** Schematic of the experiment. **D.** CRH-related events per minute. 7 mice, 1-2 observations per mice. Nested ANOVA, F_{2, 18} = 8.578, *p* = 0.002. Tukey's multiple comparison tests: T1 vs. T2, *p* = 0.002; T2 vs. T3, *p* = 0.2. **E.** Amplitude of CRH-related events. 7 mice, 1-2 observations per mice. Nested ANOVA, F_{2, 18} = 1.492, *p* = 0.3 **F.** Interaction times. 7 mice, 1-2 observations per mice. Nested ANOVA, F_{2, 18} = 5.649, *p* = 0.008. Tukey's multiple comparison tests: T1 vs. T2, *p* = 0.01; T2 vs. T3, *p* = 0.02.

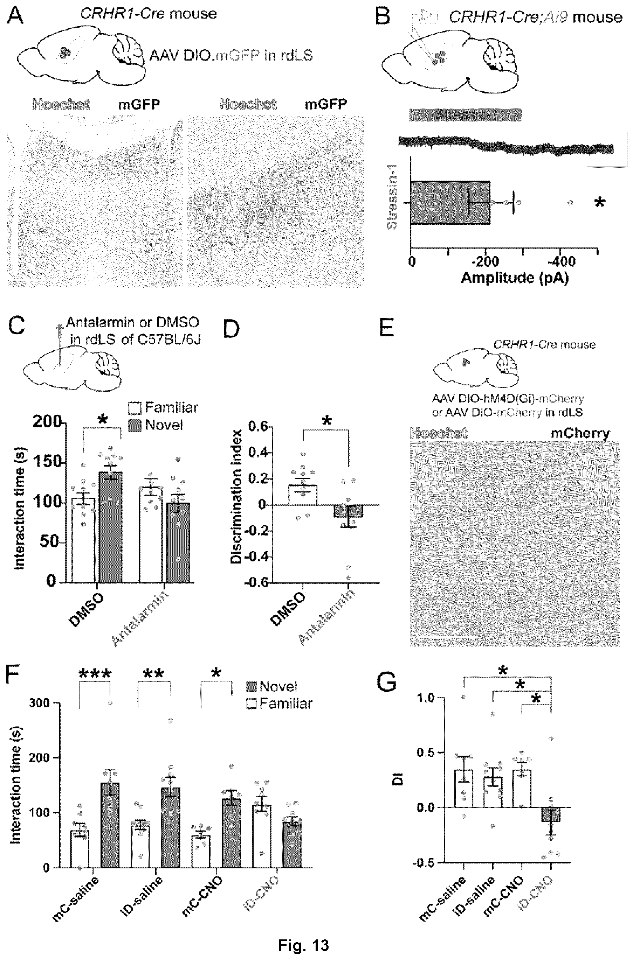

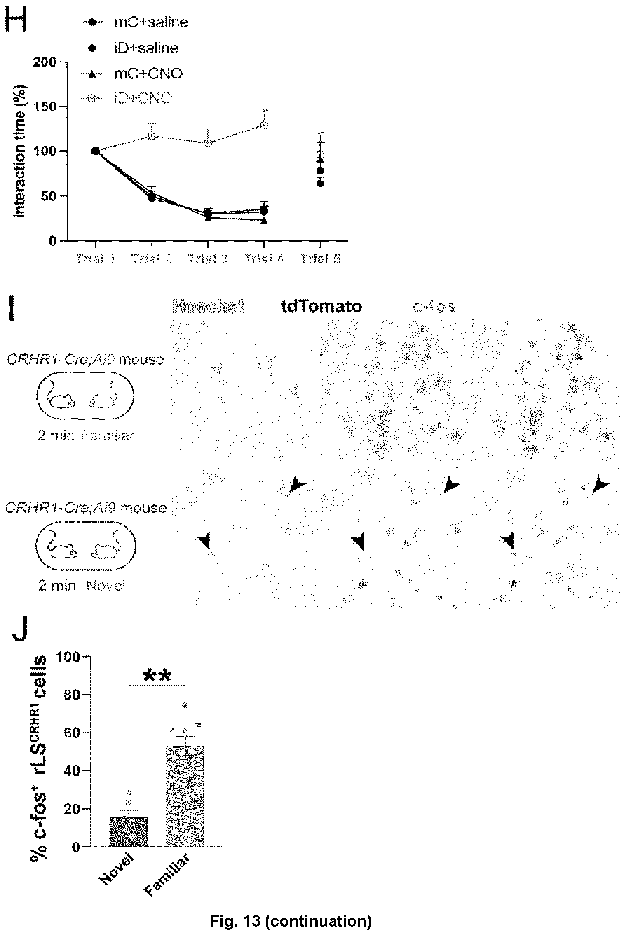

**Figure 13****. CRHR1⁺ neurons in rdLS are activated by social familiarity and regulate SNP and familiarization.**
   **A.** (Top)Scheme: *CRHR1-Cre* mice injected in rdLS with AAV2/5 hSyn.DIO.mGFP.(Botton left): Coronal section of the LS: Hoechst in light grey and CRHR1 cells expressing mGFP in black. Scale bar: 300 µm Right: Magnification of CRHR1 cells from the left panel Scale bar 50 µm . **B.** Whole-cell patch-clamp recording of CRHR1-tdTomato cells in rdLS of *CRHR1-Cre;Ai9* mice (top). Voltage-clamp trace during bath application of 300 nM stressin-1 (middle). Scale bars: 100 pA and 2 min. Bar graph showing the amplitude of the decrease (bottom). C.C57BL/6J wild-type mice infused in rostro-dorsal Lateral Septum (rdLS) with 2 µg of antalarmin dissolved in 0.6 µL of DMSO or DMSO as a control (top). Botton: Interaction time with familiar (white) or novel (dark grey) mouse during the recall trial of the social novelty preference test in mice infused with antalarmin or DMSO (bottom). Grey dots are different mice. 2-way ANOVA F(novelty x injection)_{1,36} = 7.699, *p* = 0.009. Sidak's multiple comparison tests novel vs. familiar: DMSO, *p* = 0.04; antalarmin, *p* = 0.3. D. Discrimination index for social novelty preference during recall trial. Grey dots are different mice. One-sample ttests: *p* = 0.003 and *p* = 0.2. Unpaired *t* test: *p* = 0.01. E. *CRHR1-Cre* mice injected in rdLS with AAV2/8 hSyn.DIO.hM4D(Gi)-mCherry (iDREADD) or AAV2/8 hSyn.OIO.mCherry (top). Immunohistochemistry pictures of iD-mCherry expression in rdLS. Scale bar: 300 µm (bottom) Hoechst in light grey and mCherry cells in black. **F.** Interaction time with novel (dark grey) or familiar (white) mouse during the recall trial of the social novelty preference test in mice expressing mCherry (mC) or hM4Di (iD) in saline or CNO. Grey dots are different mice. 3-way ANOVA F(novelty x injection x virus)_{1,60} = 3.845, *p* = 0.04. Sidak's multiple comparison tests novel vs. familiar: mC + saline, *p* = 0.0003; iD + saline, *p =* 0.001; mC + CNO, *p* = 0.01; iD + CNO, *p* = 0.4. G. Discrimination indexes for social novelty preference of the four groups during recall trial. One-sample t tests compared to 0: mC + saline, p = 0.02; iD + saline, *p* = 0.009; mC + CNO, *p* = 0.001; iD + CNO, *p* = 0.3. 2-way ANOVA: F(virus x injection)_{1, 30} = 4.3, *p* = 0.04; F(virus )_{1, 30} = 7.654, *p* = 0.009; F(injection)_{1, 30} = 4.263, *p* = 0.05. Tukey's multiple comparison tests compared to the iD + CNO group: mC + saline, *p* = 0.009; iD + saline, *p* = 0.02; mC + CNO, *p* = 0.01. H. Normalized interaction times during repetitive social presentations. 7-8 mice per group. Two-way ANOVA, F(trial₁₋₄ x virus)_{9,104} = 6.612, *p* < 0.0001; F(trial)_{3,104} = 28.05, *p* < 0.0001; F(virus)_{3,104} = 52.74, *p* < 0.0001. I. Immunohistochemistry pictures against c-fos of CRHR1-tdTomato mouse rdLS of following interaction with a familiar or novel mice. Scale bars: 50 µm. Grey arrowheads show that the majority of TdTomato cells express c-fos after familiar presentations. Black arrowheads show that only few TdTomato cells express c-fos after Novel presentations **J.** Percentage of CRHR1⁺ neurons expressing c-fos. 3 and 4 mice. 2 observations per mice. Nested *t* test, *p* = 0.007.

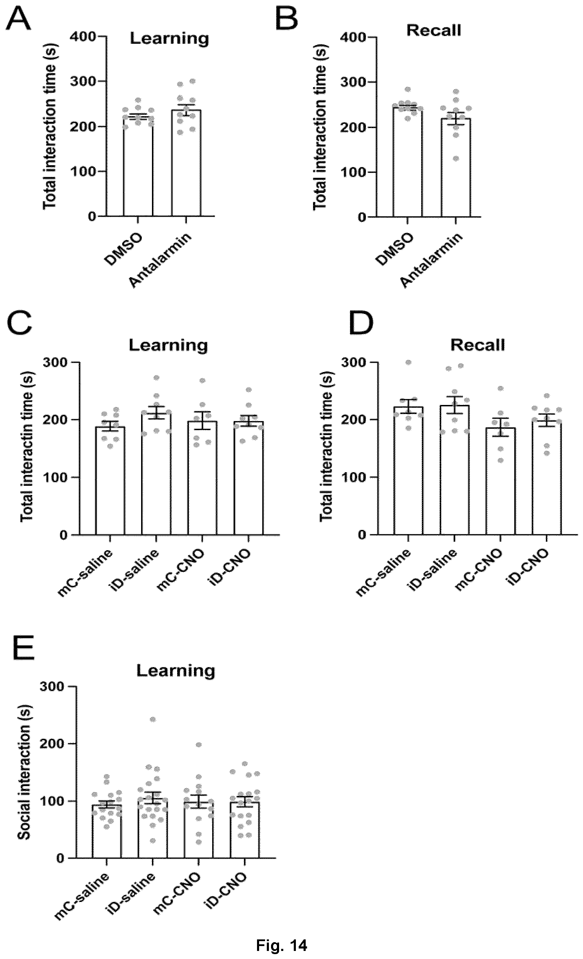

**Figure 14****. Antalarmin infusion and rdLS^{CRHR1} neurons chemogenetic silencing controls.**
   **A-B.** WT mice infused with DMSO or antalarmin. Total interaction time during the learning (A) and recall (B) phases of the social novelty preference test. **C-E.** *CRHR1-Cre* mice injected in rdLS with AAV2/8 hSyn.DIO.hM4D(Gi)-mCherry (iD) or AAV2/8 hSyn.DIO.mCherry (mC). **C-D.** Total interaction times during learning (C) or recall trial (D) of the social novelty preference test. **E.** Interaction time with each novel mouse during the learning trial of the social novelty preference test.

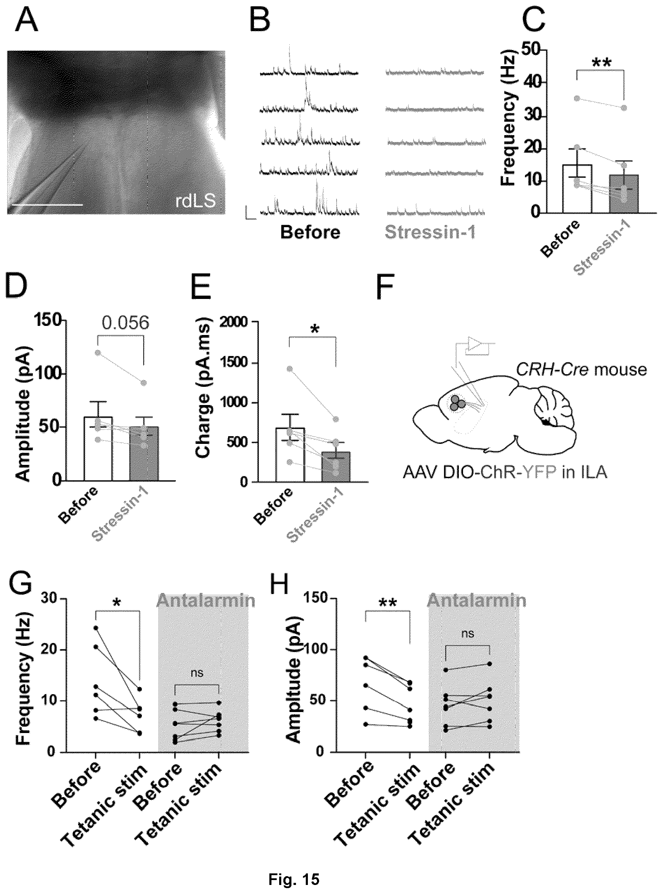

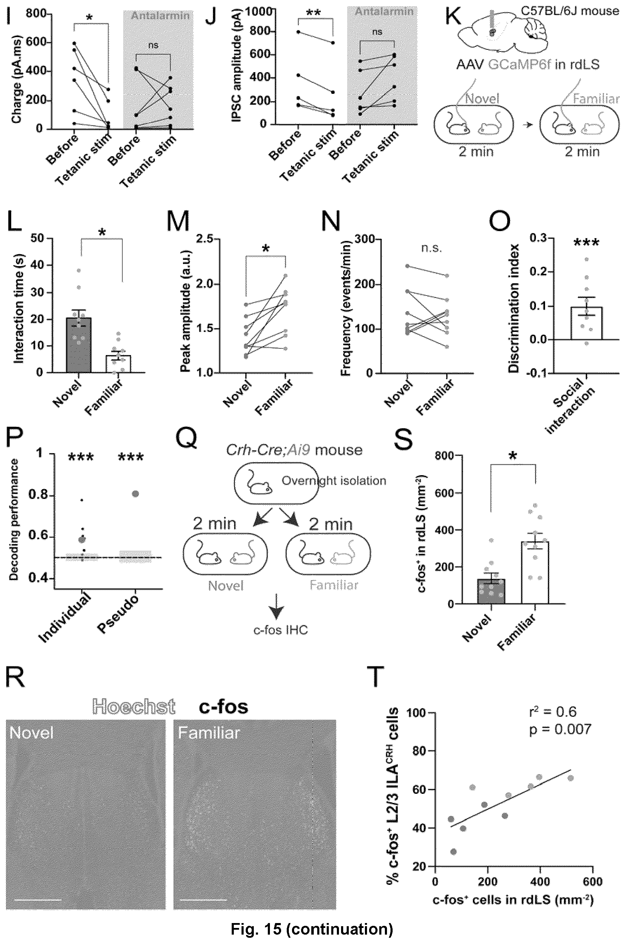

**Figure 15****. CRH signaling from ILA and familiar social interaction disinhibit rdLS.**
   **A.** Differential interference contrast microscopy image of rostro-dorsal Lateral septum (rdLS) during patch-clamp recording. Scale bar: 500 µm. **B.** Example traces of Inhibitory postsynaptic currents (IPSCs) before or 15 min after application of 300 nM stressin-1. **C.** Frequency of IPSCs. **D.** Amplitude of IPSCs. **E.** IPSCs area under the curve. For C-E, points are obtained from individual cells recorded from separate slices in 6 mice. **F.** *CRH-Cre* mice injected with AAV2/9 EF1a.DIO.hChR2(E123T/T159C)-eYFP in ILA. **G-I.** Frequency (G), amplitude (H) and charge (I) of rdLS neuron spontaneous inhibitory events before and after tetanic light stimulation with or without 300 nM antalarmin. Each observation is from a different cell in separate brain slices obtained from 6 mice and 5 mice respectively. Paired *t* tests: *p* = 0.03, 0.2, 0.0003, 0.2, 0.03 and 0.9. **J.** Electrically evoked IPSC of rdLS neuron spontaneous inhibitory events before and after tetanic light stimulation with or without 300 nM antalarmin. Paired *t* tests: *p* = 0.006 and 0.07. **K.** C57BL/6J wild-type mice injected in rdLS with AAV2/1 Syn.GCaMP6f and implanted with an optical ferrule above rdLS. Implanted mice were presented with novel then familiar mice. L. Interaction time during social presentation. 9 recording sessions using 5 mice. Nested *t* test, *p =* 0.01. **M.** Average peak amplitude of the z-score during presentation of a novel or familiar mouse. Nested *t* test: *p* = 0.03. **N.** Frequency of events during presentation of a novel or a familiar mouse. Nested *t* test: p = 0.8. O. Discrimination index for social familiarity preference calculated from z-scores. Nested t tests compared to 0: *p* = 0.002. **P.** Decoding performance for familiarity versus novelty from individual recordings or pseudo-simultaneous data. Small black dots on the left are the results from each individual recording sessions using 20 cross-validation iterations, large grey dot is the average. Big grey dot on the right is the result of pseudo-population analysis from 100 cross-validation iterations. Grey areas denote chance level computed using permutation tests (2.5-97.5 percentiles in distribution of shuffled decoding performances). In both cases, statistical significance is determined by the probability of drawing the observed decoding performance from the distribution of shuffled decoding performances (null-hypothesis). *p* < 0.001 (two-tailed permutation test, see *Methods*). Q. *CRH-Cre;Ai9* mice were presented with novel or familiar mice after overnight isolation before being processed for immunohistochemistry against c-fos. **R.** Immunohistochemistry images of c-fos labelling in rdLS following social presentation with a novel or familiar mouse (same experiment than Fig. 3N). Scale bars: 500 µm. (Hoechst in the pannels is show in dark grey and c-fos positive cells in white) **S.** Density of rdLS cells positive for c-fos. For each mouse, one observation on each side of a rLS section. 5 mice per group. Nested t test, p = 0.02. T. Percentage of layer 2/3 ILA^{CRH} cells positive for c-fos (data from. Fig. 8) vs. density of rdLS cells positive for c-fos following social interactions. Each point represents a mouse.

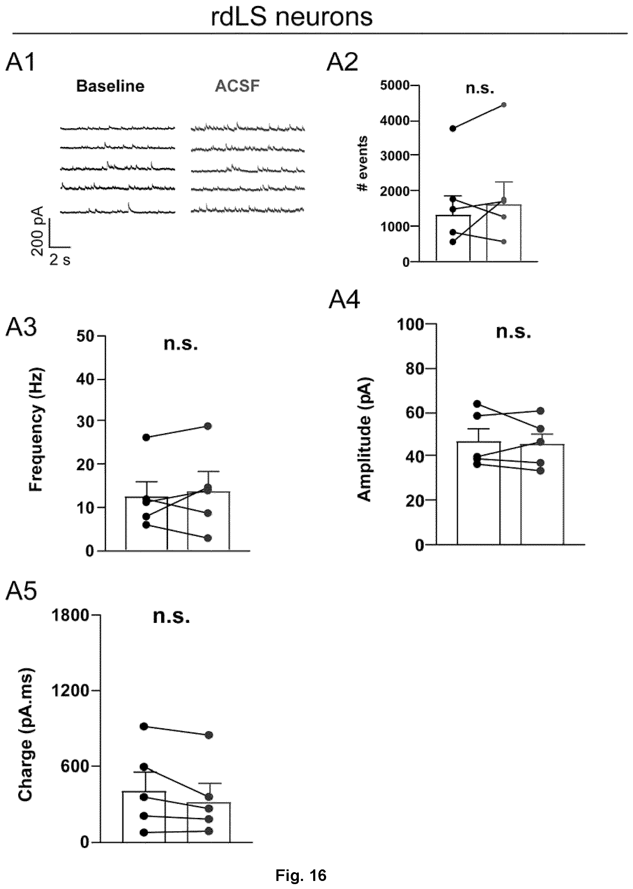

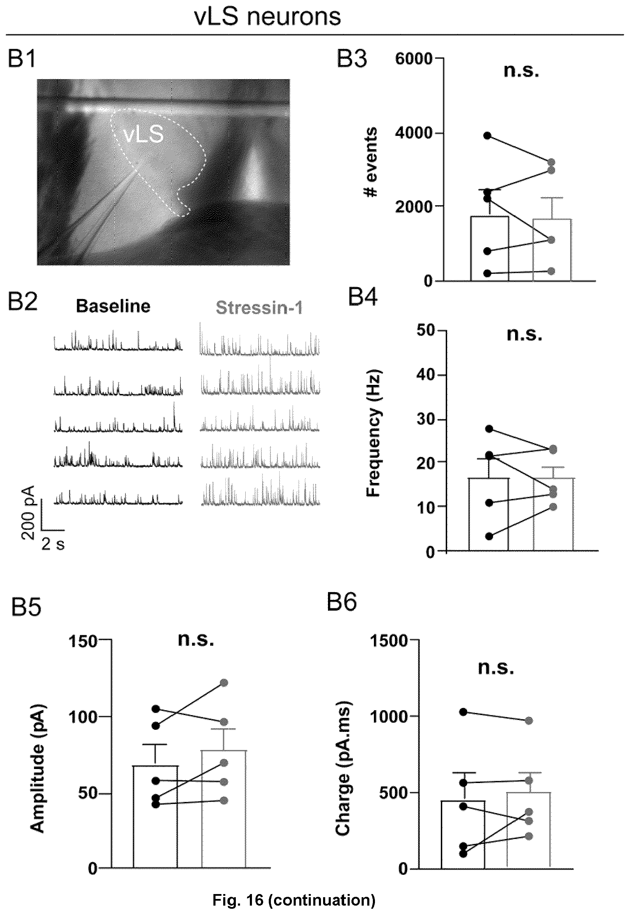

**Figure 16****. Controls for in vitro electrophysiology recordings in LS.**
   A. In vitro whole-cell patch-clamp of rdLS neurons. A1. Example trace of IPSCs before or 15 min after application of ACSF. A2. Number of IPSCs. Points are individual cells recorded in 5 mice. **A3.** Frequency of IPSCs. **A4.** Amplitude of IPSCs. **A5.** IPSCs area under the curve. **B.** Neurons recorded in vLS before and after application of 300 nM stressin-1. **B1.** DIC image of the LS region where cells were recorded. Scale bar: 200 µm. **B2.** Example trace of IPSCs before or after 15 min 300 nM stressin-1. **B3.** Number of IPSCs. Points are individual cells recorded in 5 mice. **B4.** Frequency of IPSCs. **B5.** Amplitude of IPSCs. **B6.** IPSCs area under the curve. For the entire figure, bar graphs represent mean ± S.E.M.

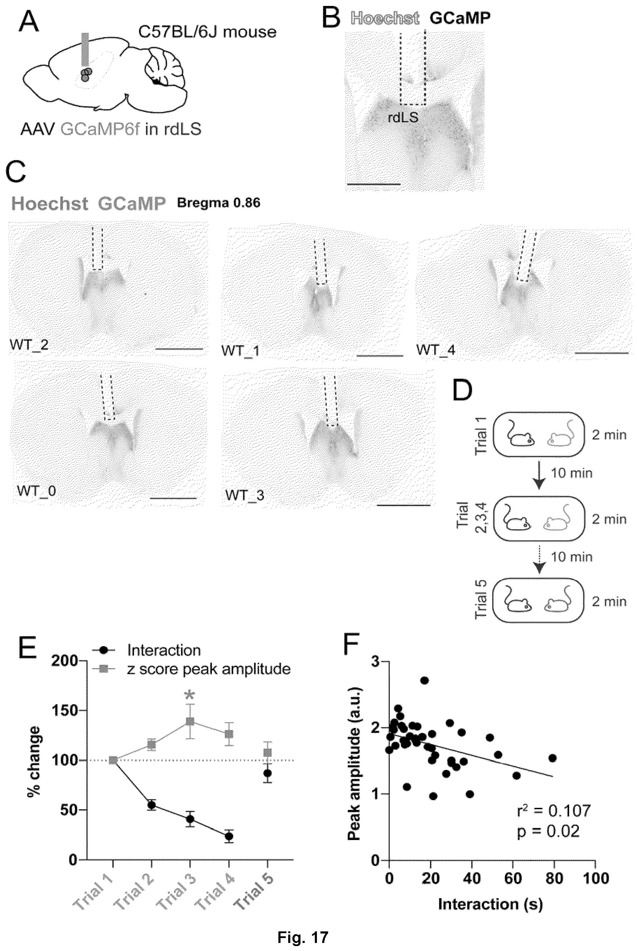

**Figure 17****. Fiber-photometry recordings in rdLS.**
   **A.** C57BL/6J wild-type mice injected in rostro-dorsal Lateral Septum (rdLS) with AAV2/1 Syn.GCaMP6f and implanted with an optical ferrule above rdLS. **B.** Immunohistochemistry image showing GCaMP (black) and Hoechst (light grey) expression in rdLS and the optical ferrule implanted above the injection site (dashed lines). Scale bar: 500 µm. **C.** Detail of every recorded mouse. Immunohistochemistry image showing GCaMP (black) and Hoechst (light grey) expression in rdLS and the optical ferrule implanted above the injection site (dashed lines). Scale bars 2 mm. **D.** Schematic of the repetitive social presentation test. **E.** Fiber-photometry recording during repetitive social presentation test (10 recording sessions in 5 mice). One-way ANOVA F(trial 1-4)_{3,27} = 3.389, *p* = 0.03 followed by Dunnett's multiple comparison tests compared to trial 1: trial 2 *p* = 0.5, trial 3 *p* = 0.01 and trial 4 *p* = 0.1. **F.** Interaction times (trial 1 to trial 4) vs. z-scores during the same experiment.

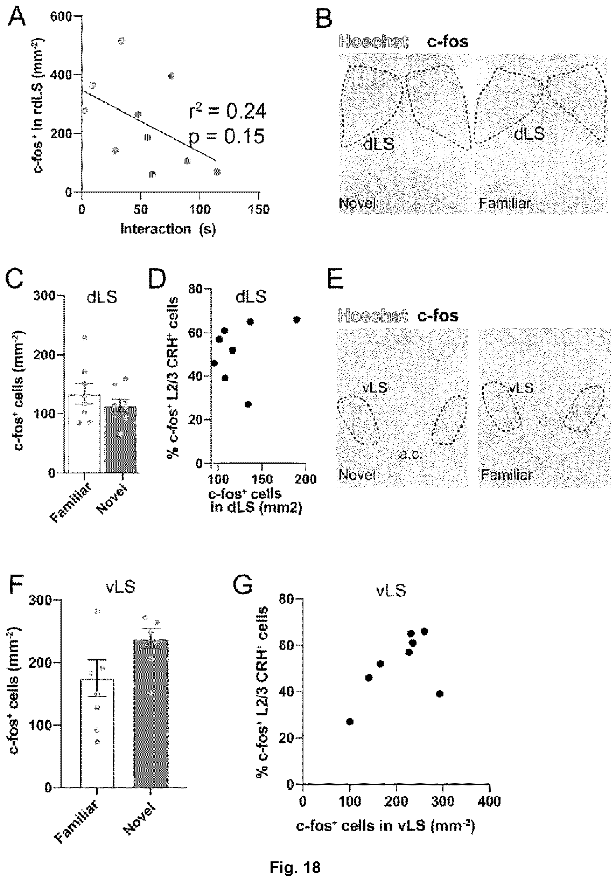

**Figure 18****. Dorsal and ventral posterior LS do not respond to social familiarity versus social novelty.**
   **A.** Density of rdLS cells positive for c-fos vs. interaction time during social interaction with novel (dark grey) or familiar (light grey) mice. Each point represents one mouse, N = 5 mice. **B.** Immunohistochemistry images of c-fos labelling in posterior dorsal LS (dLS). (Hoechst in light grey and c-fos cells in black) Scale bars: 500 µm. **C.** Density of dLS cells positive for c-fos. Each point is from a different image obtained from 4 mouse. Unpaired t test, *p* = 0.3. **D.** Percentage of ILA^{CRH} cells positive for c-fos in layer 2/3 vs. density of dLS cells positive for c-fos following social interaction. Each point represents one mouse. **E.** Immunohistochemistry images of c-fos labelling in posterior ventral LS (vLS). (Hoechst in light grey and c-fos cells in black) Scale bars: 500 µm. **F.** Density of vLS cells positive for c-fos. Each point is from a different image obtained from 4 mouse. Unpaired t test, *p* = 0.08. **G.** Percentage of ILA^{CRH} cells positive for c-fos in layer 2/3 vs. density of vLS cells positive for c-fos following social interaction. Each point represents one mouse. For the entire figure, bar graphs represent mean ± S.E.M.

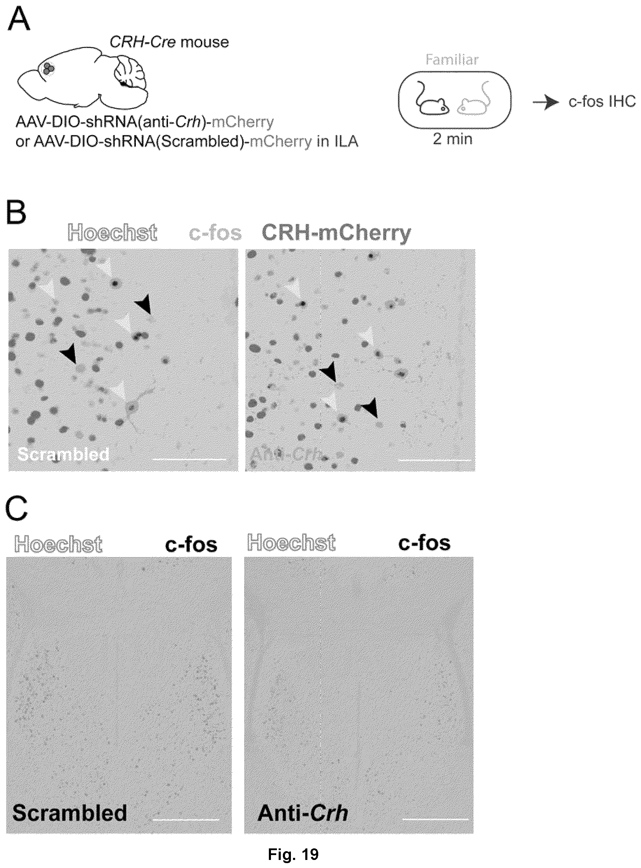

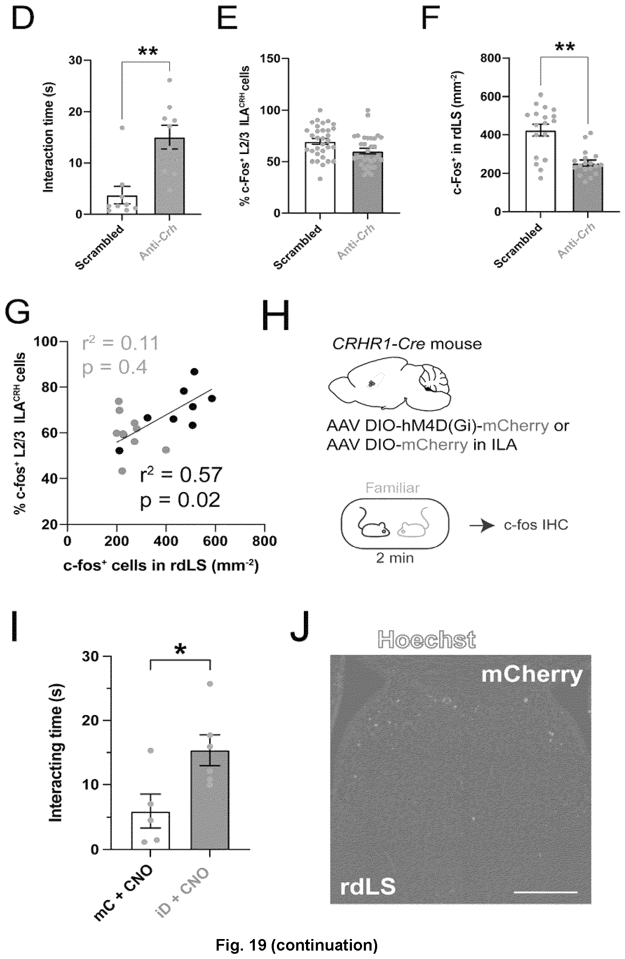

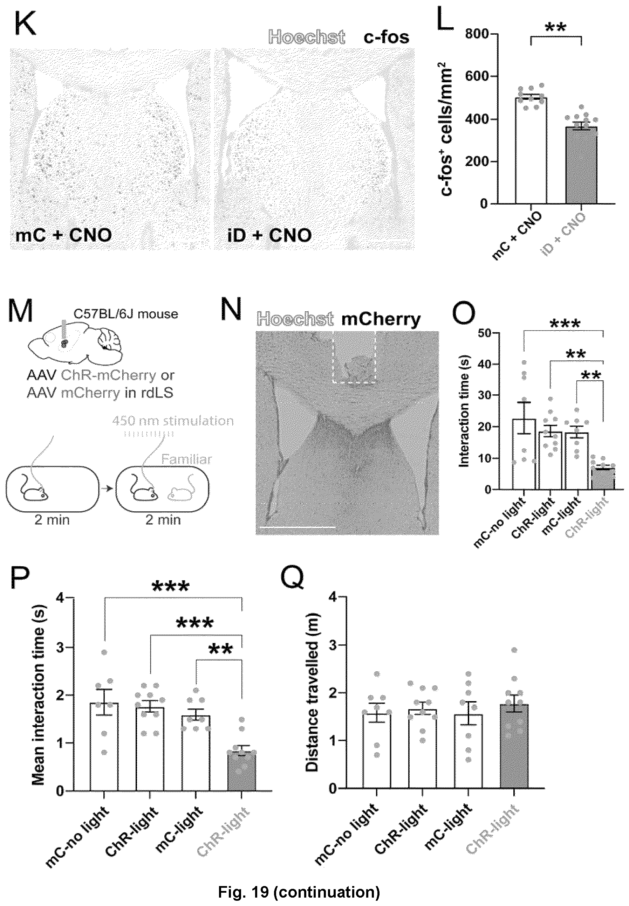

**Figure 19****. CRH release from ILA and rdLS^{CRHR1} neurons regulate rdLS disinhibition and social interaction with a familiar mouse.**
   **A.** *CRH-Cre* mice injected in ILA with AAV2/9 CMV-DIO-(mCherry-U6)-shRNA(anti-*Crh*) or AAV2/9 CMV-DIO-(mCherry-U6)-shRNA(scrambled) presented with a familiar mouse for 2 min before being processed for immunohistochemistry against c-fos. **B**lmmunohistochemistry images of c-fos labelling in infralimbic region (ILA) White arrowheads: c-fos⁺ / tdTomato⁺ cells. Black arrowheads: c-fos⁻ / tdTomato⁺ cells. Scale bars: 100 µm. C. Immunohistochemistry images of c-fos labelling in rostro-dorsal LS (rdLS) , Hoechst in light grey and c-fos cells in black Scale bar is 300 µm. **D.** Duration of interaction during familiar presentation. Each point is one mouse. Unpaired *t* test, *p* = 0.001. Scrambled virus (control) or Anti-Crh.(silencing). **E.** Percentage of layer 2/3 ILA^{CRH} cells positive for c-fos in layer 2/3 of ILA. Each point corresponds to each side of 2 sections. 9 mice per group. Scrambled virus (control) or Anti-Crh.(silencing). **F.** Density of rdLS cells positive for c-fos. One observation on each side of a rLS section. 9 mice per group. Nested *t* test, *p* = 0.002. Scrambled virus (control) or Anti-Crh.(silencing). **G.** Percentage of layer 2/3 ILA^{CRH} cells positive for c-fos vs. density of rdLS cells positive for c-fos following social interaction with a familiar mouse. Each point represents one mouse. **H.** *CRHR1-Cre* mice injected in rdLS with AAV2/8 hSyn.DIO.hM4D(Gi)-mCherry (iDREADD) or AAV2/8 hSyn.DIO.mCherry presented with a familiar mouse for 2 min before being processed for immunohistochemistry against c-fos. **I.** Interaction time with familiar mouse. Each point is one mouse. Unpaired *t* test, *p* = 0.03. Control virus (mC) and inhibitory DREADD (iD) both with intra-peritoneal injection of CNO (Clozapine N-oxide) **J.** Immunohistochemistry picture of mCherry expression in rostro-dorsal Lateral Septum (rdLS). Scale bar: 400 µm.Hoechst in dark grey and mCherry positive cells in white. **K.** Immunohistochemistry pictures of c-fos (in black) and Hoechst (light grey) expression in rdLS. Scale bar: 400 µm. **L.** Density of rdLS cells positive for c-fos. For each mouse, one observation on each side of a rLS section. 5 and 6 mice per group. Nested *t* test, *p* = 0.002. Control virus (mC) and inhibitory DREADD (iD) both with intra-peritoneal injection of CNO (Clozapine N-oxide) **M.** C57BL/6J wild-type mice were injected with AA2/2 hSyn1.hChR2(H134R)-mCherry (AAV ChR-mCherry) or AA2/2 hSyn1.mCherry as control (AAV mCherry) and an optical fiber was implanted above the injection site. Mice were then presented to a familiar mouse for 2 min meanwhile 450 nm light was applied (20 Hz, 1 ms). Mice were also run without light as additional controls. **N.** Immunohistochemistry picture of viral injection in rdLS. Hoechst in light grey and mCherry axons in black. Scale bar: 1 mm. O. Total interaction time with familiar mouse. Each point represents one mouse. One-way ANOVA: F_{3,32} = 7.01, *p* < 0.0001. Dunnett's multiple comparison tests: ChR-light vs. YFP-no light *p* = 0.0005, ChR-light vs. ChR-no light *p* = 0.006, ChR-light vs. YFP-light *p* = 0.01. **P.** Average duration of each bout of social interaction. Each point represents one mouse. One-way ANOVA: F_{3,31} = 10.62, *p* < 0.0001. Dunnett's multiple comparison tests: ChR-light vs. YFP-no light *p* = 0.0001, ChR-light vs. ChR-no light p = 0.0001, ChR-light vs. YFP-light p = 0.003. Q. Total distance travelled. Each point represents one mouse. For the entire figure, bar graphs represent mean ± S.E.M.

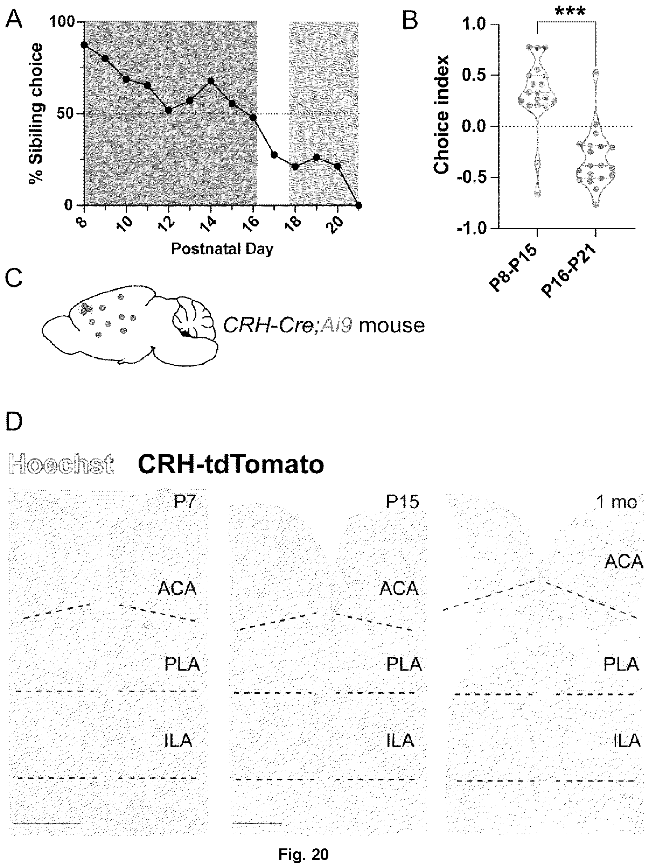

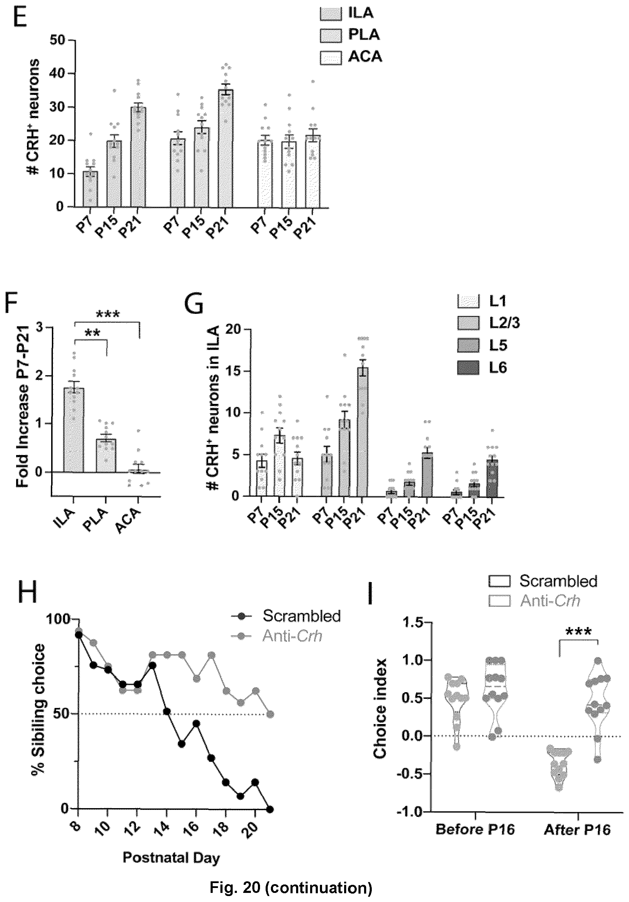

**Figure 20****. Increased CRH expression in ILA supports a shift in social preference in young mice.**
   **A.** Percentage of pups showing sibling preference (% Sibling choice) during development (postnatal days (P) 8 to 21), 19 mice. Dark square represents the temporal window for sibling preference, and light square represents the temporal window for non-sibling preference. **B.** Discrimination index for familiar kin before (P8-P15) and after postnatal day 16 (P16-P21). Each point represents a mouse, 19 mice. Unpaired *t* test, *p* < 0.001. **C.** *CRH-Cre;Ai9* mice. **D.** mPFC images of *CRH-Cre;Ai9* mice (CRH-tdTomato) at P7, P15 or 1 month in ACA, PLA and ILA. Scale bars: 500 µm. **E.** Number of CRH⁺ cells in ILA, PLA and ACA during development (P7, P15 and P21). Each point represents one observation made on each side of 2 section, 3 mice per group. Nested one-way ANOVA tests comparing CRH cells along postnatal day: F(ILA)_{2,6} = 18.64, *p* = 0.003; F(PL)_{2,6} = 11.47, *p* = 0.009; F(ACA)_{2,6} = 0.22, *p* = 0.8. F. Fold-increase of CHR⁺ cells between P7 and P21. P21 values compared to the average P7 value. Nested one-way ANOVA F_{1,6} = 63.03, *p* < 0.0001. Post-hoc Tukey's multiple comparison test: ILA vs. PLA *p* = 0.001; ILA vs. ACA *p* < 0.0001. G. Number of CRH⁺ cells per ILA layers during development (P7, P15 and P21). Each point represents one observation made on each side of 2 section, 3 mice per group. H. Percentage of familiar choice during development in *CRH-Cre* mice injected in ILA with AAV2/9 CMV-DIO-(mCherry-U6)-shRNA (anti-*Crh*) to downregulate *Crh* or control AAV2/9 CMV-DIO-(mCherry-U6)-shRNA (scrambled). 12 pups per group. Chisquare test: *p* < 0.0001. I. Discrimination index for familiar kin before and after postnatal day 16. Each point represents a mouse, 12 pups per group. Unpaired *t* tests: p = 0.3 and *p* < 0.0001. For the entire figure, bar graphs represent mean ± S.E.M. P7:Postnatal day 7, P15: Postnatal day 15, 1mo: 1 month, ACA: Anterior cingulate area, PLA: Prelimbic area, ILA: Infralimbic area. Dashed lines represent the limit between regions in the medial prefrontal cortex.

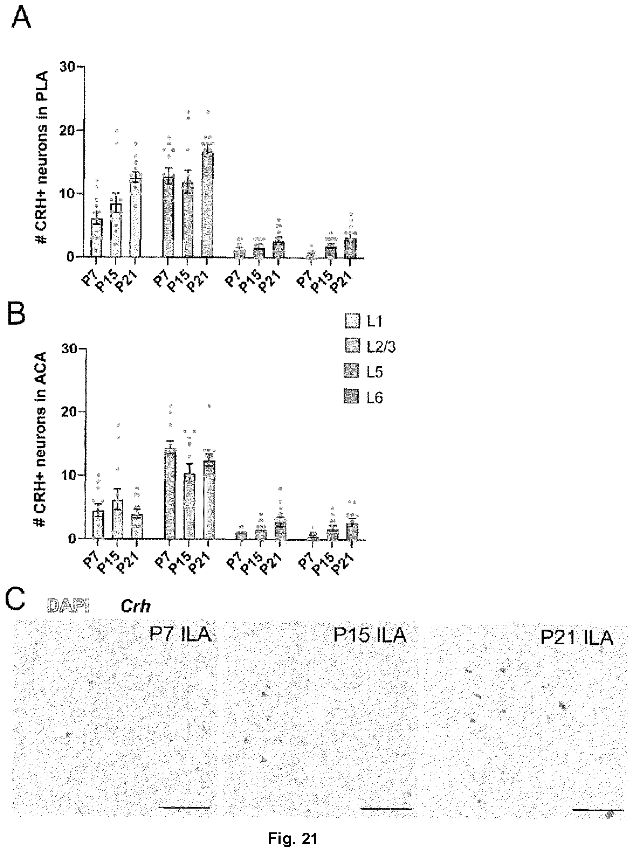

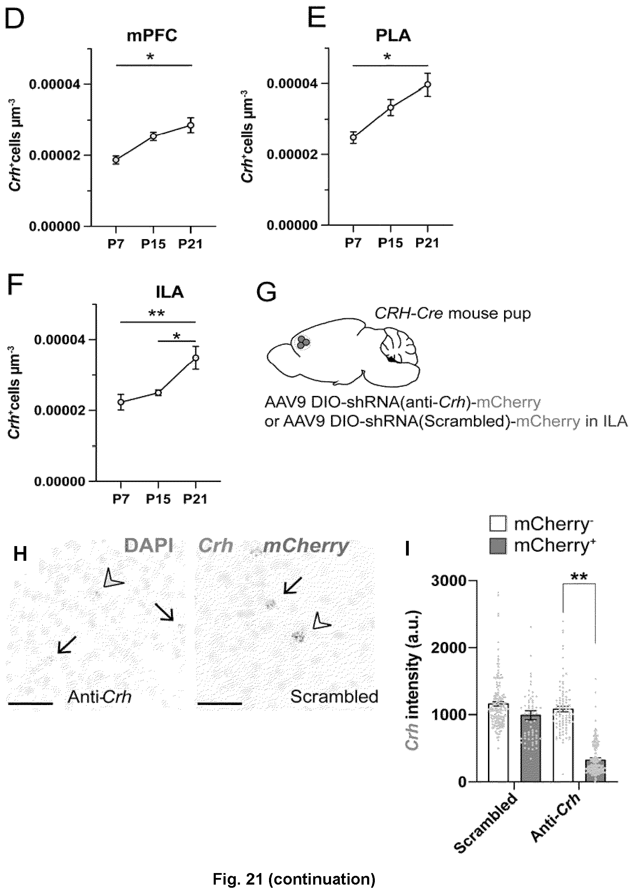

**Figure 21****. CRH⁺ cell distribution per layers in PLA and ACA, *Crh* expression in ILA across ages and *Crh* knock-down in young pups.**
   **A.** Evolution of the number of CRH⁺ cells per layer on the Prelimbic area of the prefrontal cortex (PLA). CRH+ cells were quantified from mPFC images of *CRH-Cre;Ai9* mice (CRH-tdTomato) at P7, P15 or P21. **B.** Evolution of the number of CRH⁺ cells per layer on the Anterior cingulate area of the prefrontal cortex (ACA). CRH+ cells were quantified from mPFC images of *CRH-Cre;Ai9* mice (CRH-tdTomato) at P7, P15 or P21. Each point represents one observation made on each side of 2 section, 3 mice per group.L1: Layer 1 of the cortex, L2/3: Layer 2/3 of the cortex, L5: Layer 5 of the cortex, L6: Layer 6 of the cortex. **C.** In situ hybridization pictures against *Crh* of Infralimbic area (ILA) at Postnatal day (P)7, P15 and P21. Dapi in light grey and *Crh* in black. Scale bars 100 µm. **D-F.** Density of *Crh*⁺ cells in medial prefrontal cortex (mPFC) (D), PLA (E) and ILA (F). 3 mice per age. Nested one-way ANOVAs followed by Tukey's multiple comparison tests. F_{2,28} = 6.731, p = 0.03; F_{2,28} = 8.088, p = 0.02; F_{2,27} = 7.766, p = 0.002. **G.** *CRH-Cre* pup mice injected in ILA with AAV2/9 CMV-DIO-(mCherry-U6)-shRNA(anti-*Crh*) to downregulate *Crh* or control AAV2/9 CMV-DI0-(mCherry-U6)-shRNA(scrambled). **(H)** In situ hybridization images of ILA slices expressing shRNA against *Crh* (left) or a scrambled shRNA (right) labelled for *mCherry* and *Crh.* White arrows denote CRH⁺ neurons that do not express virus. Grey arrowhead denotes CRH⁺ cells expressing the anti-Crh shRNA, with reduced level of *Crh.* White arrowheads denote CRH⁺ neurons expressing the scrambled shRNA, with intact *Crh* level. Black arrows denote mCherry negative cells (no virus) with intact levels of *Crh.* Scale bars: 50 µm. **I.** Quantification of *Crh* expression in cells using in situ hybridization images in ILA slices from mice injected with AAV expressing scrambled or anti-*Crh* shRNAs. In each slice neurons were classified as to whether they were uninfected or infected with virus based on *mCherry* expression (3 mice per group; each point is from a different neuron). Note the reduction in *Crh* expression in neurons infected with anti-*Crh* shRNA. Nested one-way ANOVA F_{3,8} = 16.44, *p =* 0.0009 followed by Tukey's multiple comparison test, anti-*Crh* + mCherry⁺ vs. anti-*Crh* + mCherry⁻, *p =* 0.0012. Bar graphs represent mean ± S.E.M.

### EXAMPLES

### MATERIALS AND METHOD

### Experimental models and subject details

All animal procedures were performed in accordance with the regulations of the UMH-CSIC IACUCs. P5 to 16-week-old C57BL6/J wild-type (Jackson Laboratories, #000664) mice were used, as well as mice of the same age range from the following transgenic mouse lines: *CRH-Cre* mice (Jackson Laboratories, #012704) and *CRHR1-Cre* mice (courtesy of Jan Deussing). *CHR-Cre* were crossed to the *Ai9* tdTomato Cre-reporter mice (Jackson Laboratories, #007909) in order to visualize CRH⁺ neurons. All transgenic mice were on the C57BL6/J background. During social interaction tests, stimulus mice were C57BL6/J wild-type mice of the same gender and age than the test mouse. No difference was observed related to sex and the results were pooled together. Below is a table (Table 1) summarizing number of male and female mice used in each behavioral experiment.

**Table 1. Number of male and female mice used in each behavioral experiment.**

| **EXPERIMENT** | **Male** | **Female** |
|---|---|---|
| CRH-iD SNP | 30 | 28 |
| CRH-iD repetitive social presentation | 8 | 8 |
| CRH-eD repetitive social presentation | 8 | 8 |
| CRH fiberphotometry single presentation | 5 | 0 |
| CRH fiberphotometry repetitive social presentation | 5 | 0 |
| CRH-tdT c-fos of ILA and rdLS | 5 | 5 |
| shRNA anti-Crh repetitive social presentation | 4 | 4 |
| shRNA anti-Crh SNP | 8 | 8 |
| CRH biosensor | 8 | 0 |
| CRH-Arch SNP | 15 | 0 |
| CRH-Arch repetitive social presentation | 11 | 0 |
| WT antalarmin infusion SNP | 10 | 10 |
| CRHR1-iD SNP | 15 | 10 |
| CRHR1-iD repetitive social presentation | 15 | 15 |
| CRHR1-iD c-fos of LS single presentation | 7 | 4 |
| WT Fiberphotometry in rLS single presentation | 5 | 0 |
| CRH-tdT c-fos of ILA and rdLS following single presentations | 5 | 5 |
| CRH-shRNA anti-Crh c-fos after familiar presentation | 14 | 4 |
| CRHR1-iD c-fos after familiar presentation | 7 | 5 |
| WT ChR in LS | 17 | 0 |
| Kinship in WTs | 10 | 9 |
| Kinship in with anti-Crh shRNA | 15 | 9 |
| CRH-iD Open Arena | 11 | 11 |
| CRH-iD elevated plus maze | 18 | 11 |
| CRH-iD Feeding | 15 | 4 |
| Sociability iD CRH-ILA | 20 | 10 |
| Repetitive Trial (saline) iD CRH-ILA | 8 | 8 |
| Repetitive Trial (saline) eD CRH-ILA | 8 | 8 |
| CRH-iD and CRH-eD c-fos control single presentation | 5 | 7 |
| CRH-iD novel object recognition | 15 | 4 |
| CRH-iD repetitive object presentation | 4 | 4 |
| CRH-iD fod preference | 5 | 6 |
| CRH-GCaMP fiberphotometry during object presentations | 3 | 0 |
| shRNA anti-vGAT repetitive social presentation | 10 | 7 |
| shRNA anti-vGAT SNP | 15 | 7 |
| retrograde shRNA anti-CRH repetitive social presentation | 15 | 0 |
| retrograde shRNA anti-CRH repetitive social presentation | 10 | 0 |
| CRH-Arch + biosensor in LS during familiar presentation | 7 | 0 |
| WT rLS fiberphotometry repetitive social presentation | 5 | 0 |
| c-fos in dLS following single presentations | 5 | 5 |

### Anti-Crh shRNA design and in vitro validation

Three different shRNAs that target *Crh* (shRNA1: GCCCTTGAATTTCTTGCAGCC (SEQ ID NO: 2); shRNA2: GCATGGGTGAAGAATACTTCC (SEQ ID NO: 3); shRNA3: GGAAACTGATGGAGATTATCG (SEQ ID NO: 4)) were cloned into the PX552 plasmid (Addgene #60958) to make rAAV-U6-shRNA1,2,3(CRH)-CMV-EGFP-SV40-polyA (Brain VTA #PT-2784, #PT-2785 and #PT-2786). In addition, a scrambled shRNA control was cloned into the same plasmid to make rAAV-U6-shRNA(scrambled)-CMV-EGFP-SV40-polyA (Brain VTA #PT-0916). To overexpress CRH mRNA, the sequence for mouse CRH was cloned into a plasmid to construct rAAV-CMV-CRH-P2A-EGFP-WPRE-hGH-polyA (Brain VTA #PT-2827). For validation, HEK293T cells were transfected, in triplicate, with one of the 3 plasmids containing anti-Crh shRNAs or the scrambled shRNA construct along with the overexpression plasmid for *Crh* (CMV-CRH-GFP). The cells were collected 48hr post-transfection and RNA was purified (MiniBEST Universal RNA Extraction Kit; Takara , 9767) and subjected to RT-PCR (One Step SYBR^{®}PrimeScript^{™}RT-PCR Kit II; Takara, RR086A) using primers for CRH (F: CCCCGCAGCCCTTGAATTTCTTG (SEQ ID NO: 5); R: GGGCGTGGAGTTGGGGGACAG (SEQ ID NO: 6)) and GAPDH (F: GCAAATTCCATGGCACCGTCAAGG (SEQ ID NO: 7); R: CGCCAGCATCGCCCCACTTG (SEQ ID NO: 8)) as a control.

The RT-PCR results revealed that all three of the anti-*Crh* shRNAs showed robust decreases of *Crh* mRNA relative to the scrambled shRNA control. The anti-*Crh* shRNA-2 showed the highest knockdown efficiency and was selected for *in vivo* knockdown experiments. The anti-*Crh* shRNA-2 and the scrambled shRNA were cloned into a Cre-dependent plasmid (Botta, P., et al. (2015). Regulating anxiety with extrasynaptic inhibition. Nature Neuroscience 2015 18:10 18, 1493-1500) to make rAAV-CMV-DIO-(mCherry-U6)-shRNA(*anti-Crh*)-WPRE-hGH-polyA (Brain VTA, #PT-2787) and rAAV-CMV-DIO-(mCherry-U6)-shRNA(scrambled)-WPRE-hGH-polyA (Brain VTA, #PT-2788) which were subsequently packaged into AAV9.

### Virus injections

For all injections, animals were anesthetized using isoflurane and given analgesics. A craniotomy was performed above the target region and a glass pipette was stereotaxically lowered down the desired depth. Injections were performed using a nano-inject II (Drummond Scientific). 23nL were delivered 10s apart until the total amount was reached. The pipette was retracted after 5 min. With homozygous animals (C57BU6J wild-type or *CRH-Cre* mice), injection of the virus injection expressing DREADD, ArchT, shRNA(anti-*Crh*) and their control viruses (fluorophore only) was randomized within each cage.

### AA Vs injections in ILA

Injection coordinates were the following (in mm from Bregma): AP: 1.65, ML: ±0.1, DV: -2.6. Injections were done bilaterally with 100 nl injected per site. AAV2/DJ hSyn.FLEX.mGFP.2A.Synatophysin-mRuby (Addgene #71760 prepared by the Stanford University vector core #1930), AAV2/8 hSyn.DIO.hM4D(Gi)-mCherry (Addgene #44362-AAV8), AAV2/8 hSyn.DIO.mCherry (Addgene #50459-AAV8), AAV2/5-hSyn-DIO-hM3D(Gq)-mCherry (Addgene, #44361-AAV5), AAV2/5 hsyn.DIO.mCherry (Addgene #50459-AAV5), AAV2/1 syn.FLEX.GCaMP6f.WPRE.SV40 (Addgene #100833-AAV1), AAV2/9 CMV-DIO-(mCherry-U6)-shRNA(anti-*Crh*) (VTA brain), AAV2/9 CMV-DIO-(mCherry-U6)-shRNA(scrambled) (VTA brain), AAV2/9 hSyn.FLEX.dsRed-shRNA(Vgat) (Addgene #67845), AAV 2/9 hSyn-flex-dsRed-shRNA(scrambled) (Addgene #71383), AAV2/2 CAG.FLEX.ArchT-tdTomato (Addgene #28305 prepared by the University of North Carolina vector core) and AAV2/2 CAG.FLEX.tdTomato (Addgene #28306 prepared by the University of North Carolina vector core) were injected into the ILA of *CRH-Cre* mice. Viruses expressed for a minimum of 2 weeks.

### ACA and PLA anterograde tracing injections

AAV2/DJ hSyn.FLEX.mGFP.2A.Synatophysin-mRuby (Stanford vector core #1930 /Addgene #71760A) was injected in ACA and PLA of *CRH-Cre* mice. Injections were done unilaterally with 100 nl injected per site. Injection coordinates were the following (in mm from Bregma): PLA: 1.65, ML: ±0.1, DV: -1.4 and ACA 1.65, ML: ±0.1, DV: -0.6. Viruses expressed for a minimum of 2 weeks.

### AAV injections in rdLS

Unless specified otherwise, viruses were injected at the following coordinates in mm from Bregma: AP: 0.9, ML: ±0.2, DV: -2.8. 100 nL of HSV hEF1a.LSIL.mCherry, (Rachel Neve, Massachusetts General Hospital #RN406) was injected bilaterally into the rdLS of *CRH-Cre* mice. 100 nL of AAV2/5 DIO.mGFP (University of North Carolina, #AV4310i) was injected unilaterally into *CRHR1-Cre* mice. 200 nL of AAV2/1 syn.GCaMP6f.WPRE.SV40 (University of Pennsylvania, #AV-1-PV2822) or AAV2/9 syn.CRF1.0 (Yulong Li Lab, Wang et al., 2022) was injected unilaterally into C57BL/6J WT mice. 100 nL of AA2/2 hSyn1.hChR2(H134R)-mCherry.WPRE (University of Zurich #V-124) or AA2/2 hSyn1.mCherry.WPRE (Addgene #114472-AAV2) was injected bilaterally into C57BL/6J WT mice. 100 nL of AAV2/8 hSyn.DIO.hM4D(Gi)-mCherry (Addgene #44362) or AAV2/8 hSyn.OIO.mCherry (Addgene #50459) was injected bilaterally into the rdLS of *CRHR1-Cre mice.* All viruses expressed for a minimum of 2 weeks.

### Dual injection in rdLS and ILA

C57BU6 mice was injected with 200 nL of HSV hEF1a.Cre in rdLS (coordinates in mm from Bregma: AP: 0.9, ML: 0, DV: -2.8). 100 nL of AAV2/9 CMV-DIO-(mCherry-U6)-shRNA(anti-*Crh*) or AAV2/9 CMV-DIO-(mCherry-U6)-shRNA(scrambled) was injected bilaterally in ILA (coordinates in mm from Bregma): AP: 1.65, ML: ±0.1, DV: -2.6). 3 weeks for viral expression was waited.

### Optical ferrule implants

Animals were anesthetized using isoflurane and given analgesics. The scalp was removed and the inventors applied Vetbond^{™} (3M^{™} #7000002814) along the cut. A craniotomy was performed above the target region and the optical ferrule was lowered until the desired depth. Superglue was applied to hold the lens in position and then dental cement (GC FujiCEM 2) was applied to cover the exposed skull and keep the optical ferrule in position. Animal were allowed to recovered for 5 days before being used.
- For fiberphotometry recording of ILA^{CRH} cells in the right hemisphere the inventors implanted the optical ferrule (B280-4419-3, Doric) at the following coordinates: AP: 1.65, ML: 0.1, DV: -2.4.
- For fiberphotometry recording of rdLS cells the inventors implanted the optical ferrule at the following coordinates: AP: 0.9, ML: 0, DV: -2.65.
- For silencing of ILA^{CRH} cells fibers in rdLS cells the inventors implanted optical ferrules (Thorlab # FT200UMT and # CFLC230-10) bilaterally at the following coordinates: AP: 0.9, ML: ±0, DV: -2.65.
- For excitation rdLS cells the inventors implanted the optical ferrule (Thorlab # FT200UMT and # CFLC230-10) at the following coordinates: AP: 0.9, ML: 0, DV: - 2.65

### Cannula guide implantation and micro-infusion

The mouse scalp was removed and scored before a hole was drilled (AP +0.9, ML ±0). A cannula guide extending 2.4 mm below the pedestal (Plastics One #C315G 2-G11-SPC) was lowered slowly and kept in place using superglue. The skull was then covered with dental cement (GC FujiCEM 2) and dummy cannulas (Plastics One #C315DC-SPC) were inserted into the guides. The mice were returned to their home cages and left to recover for at least 1 week. For rdLS infusion, mice were placed under light isoflurane anesthesia (2%) and the dummy cannula was removed. A cannula (Plastics One #C315I-SPC) projecting 2.5 mm from the tip of the cannula guide was mounted. 0.6 µL of a solution containing 2 µg of antalarmin dissolved in DMSO or DMSO (Sigma-Aldrich # D8418) only were infused over 5 min using the Fusion 200 syringe pusher (Chemix Inc.) mounted with a 2-µl syringe (Hamilton #88511). The cannula was removed 2 min after the end of the micro-infusion. Mice typically recovered fully from the light anesthesia within 5 min. Mice were returned to their home cages 20 min before the test began.

### Immunohistochemistry (IHC)

Mice were anesthetized using isoflurane then perfused in the heart with 10 mL saline and their brains were quickly extracted and incubated in 4% PFA overnight. After 1 h washing in PBS, 60 µm slices were prepared using a Leica VT1000S vibratome (Leica Biosystems). Unless indicated otherwise, slices were permeabilized for 2h in PBS with 0.5% Triton-X100 (T9284, Sigma-Aldrich) in PBS before being incubated overnight at 4°C with primary antibodies diluted in PBS with 0.5% Triton-X in PBS. The slices were washed in PBS for 1 h then incubated overnight at 4°C with secondary antibodies from Thermo-Fisher Scientific at a concentration of 1:500 diluted in PBS with 0.1% Triton-X. Hoechst counterstain was applied (Hoechst 33342 at 1:1000 for 30 min in PBS at RT) prior to mounting the slice using fluoromount (Sigma-Aldrich). Images were acquired using inverted confocal microscopes (Ism 900, Zeiss and SPII, Leica) or an epifluorescent microscope (Thunder, Leica). For post-hoc immunocytochemistry after patch-clamp recordings, slices were fixed for 1 h in PBS with 4% PFA and streptavidin was applied during secondary incubation.
- For mRuby and GFP labelling, primary incubation was performed overnight at 4°C with rabbit anti-RFP (1:500, Rockland Antibody, #600-401-379) and chicken anti-GFP (1:1000, Aves, #GFP-1020) antibodies. Secondary incubation was performed with anti-rabbit antibody conjugated to Alexa 568 (#A11036) and anti-chicken antibody conjugated to 488 (#A11039).
- For YFP, GFP, GCaMP or CRF1.0 labelling, incubation was performed overnight at 4°C with anti-GFP antibody conjugated to Alexa 488 (1:500, Thermo-Fisher Scientific #A21311). No immunohistochemistry was performed against mCherry.
- For GABA labelling, incubation was performed during 2 days at 4°C with an anti-GABA antibody (1:200, Abcam, #ab17413). Secondary incubation was performed with anti-guinea-pig antibody conjugated to Alexa 647 (#A21450). No immunohistochemistry was performed against GFP.
- For mCherry labelling, primary incubation was performed overnight at 4°C with an anti-RFP antibody (1:500, Rockland Antibody, #600-401-379). Secondary incubation was performed with anti-rabbit antibody conjugated to Alexa 568 (#A11036).
- For c-fos labelling, primary incubation was performed overnight at 4°C with anti-c-fos antibody (1:1000, Abcam, #ab190289). Secondary incubation was performed with an anti-rabbit antibody conjugated to Alexa 568 (#A11036).
- For c-fos labelling, primary incubation was performed overnight at 4°C with an anti-c-fos antibody (1:1000, Abcam, #ab190289). Secondary incubation was performed with anti-rabbit antibody conjugated to Alexa 647 (#A32733). No immunohistochemistry was performed against mCherry or tdTomato.
- For tdTomato or mCherry labelling, primary incubation was performed overnight at 4°C with an anti-RFP antibody (1:500, Rockland Antibody, 600-401-379). Secondary incubation was performed with anti-rabbit antibody conjugated to Alexa 568 (#A11036).
- For tdTomato and CRF1.0 labelling, primary incubation was performed overnight at 4°C with an anti-RFP antibody (1:500, Rockland Antibody, 600-401-379). Secondary incubation was performed with anti-rabbit antibody conjugated to Alexa 568 (#A11036) and anti-GFP antibody conjugated to Alexa 488 (1:500, Thermo-Fisher Scientific #A21311).
- For GFP or GCaMP6f labelling, incubation was performed overnight at 4°C with an anti-GFP antibody conjugated to Alexa 488 (1:500, Thermo-Fisher Scientific, #A21311).
- For c-fos and mCherry labelling, primary incubation was performed overnight at 4°C with an anti-c-fos antibody (1:1000, Abcam, #ab190289) and anti-mCherry antibody (1:1000, Biorbyt, #orb153320). Secondary incubation was performed with anti-rabbit antibody conjugated to Alexa 647 (#A32733) and anti-goat secondary antibody conjugated to Alexa 568 (#A11057).

*Fluorescence quantification in CRH-Cre mice injected in the PFC with AAV2*/*DJ hSyn.FLEX.mGFP.2A.Synatophysin-mRuby:* Images were acquired using an epifluorescent microscope (Thunder, Leica). Images at 8 bits (0 to 255 intensity units/pixel) were analyzed using the software Image J. For each picture, we measure the mean fluorescence value of the background and then subtracted it from the mean fluorescence value of the region of interest (ROI). Fluorescence intensity = (Mean fluorescence of ROI - Mean fluorescence of the background).

### In situ hybridization (ISH)

Mice were anesthetized using isoflurane then decapitated and their brain quickly extracted. Brains were then immersed in dry-ice cold Butan X for 6 s before being stored at -80°C. 16 µm-thick slices were prepared using a Leica cryostat (CM3050 S, Leica Biosystems) and mounted on *Superfrost Plus* microscope slides (12-550-15, FisherBrand). For *Crh* and *mCherry* labelling, the inventors processed the slices using the RNAscope Multiplex Fluorescent Detection kit v1 (ACD Bio, #320851) with the probes for *Crh* in C1 (#316091), *mCherry* in C2 (#431201-C2). The inventors applied Protease IV for 30 s and used the Amp4 Alt-A color module. The first version of the kit was discontinued and the inventors performed the *Crh* only labelling using the RNAscope Multiplex Fluorescent Detection kit v2 (#323110) with *Crh* in C1 (#316091) with 30 s of Protease IV for and used the TSA Vivid Dye 520 at 1:750. The inventors also performed *Vgat*/*dsRed* labelling using the RNAscope Multiplex Fluorescent Detection kit v2 (ACD Bio, #323110) *vGAT* in C1 (#319191) and *dsRed* in C2 (#481361-C2) with 2 min of Protease IV. Fluorescent dyes TSA Vivid Dye 520 at 1:750 for *vGAT* and TSA Vivid Dye 650 at 1:750 for *dsRed* were used. DAPI was apply for 30 s prior to mounting using fluoromount. Images were acquired using a confocal microscope (SPII, Leica).

### In vitro electrophysiological recordings

The inventors prepared coronal brain slices from 8- to 12-week-old C57BL6/J mice. Animals were killed under isoflurane anesthesia by perfusion into the right ventricle of ice-cold solution containing the following (in mM): 125 NaCl, 2.5 KCI, 22.5 glucose, 25 NaHCOs, 1.25 NaH₂PO₄, 3 Na Pyruvate, 1 Ascorbic acid, 2 CaCl₂ and 1 MgCl₂). ACSF was saturated with 95% O₂ and 5% CO₂, pH 7.4. Brains were cut into 400 µm slices with a vibratome (VT1200S, Leica) in the same ice-cold dissection solution. Slices were then transferred to an incubation chamber containing the same ACSF solution. The chamber was kept at 34°C for 30 min and then at room temperature for at least 1 h before recording. All experiments were performed at room temperature. Slices were mounted in the recording chamber under a microscope. Recordings were acquired using the Multiclamp 700A amplifier (Molecular Device), data acquisition interface ITC-18 (Instrutech) and the Axograph software. The inventors targeted CA2 PNs based somatic location and size in both deep and superficial layer. Whole-cell recordings were obtained from LS neurons in voltage-clamp mode at -70 mV patch pipette (3-5 MΩ) containing the following (in mM): 135 Cs-gluconate, 5 KCI, 0.2 EGTA-Na, 10 HEPES, 2 NaCl, 5 ATP, 0.4 GTP, 10 phosphocreatine, and 5 µM biocytin, pH 7.2 (280-290 mOsm). The liquid junction potential was 1.2 mV and was left uncorrected. Inhibitory currents were recorded in voltage-clamp configuration at +10 mV. The inventors recorded rdLS neurons in septal slices from *CRH-Cre* mice expressing channelrhodopsin and applied 2 µM SR 95531 (Tocris, # 1262) and 1 µM CGP 55845 (Tocris, #1248) to block GABA_{A} and GABA_{B} receptors, respectively while monitoring the light induced IPSCs in rdLS neurons. The inventors also applied a tetanic light stimulation (3 times 100 pulses of 1 ms at 100 Hz) with or without 300 nM antalarmin (Tocris, #2778) present in the bath. LS neurons from WT mice were also recorded. After 10 min of stable baseline recording, stressin-1 (300 nM, Tocris # 1608) was applied following a 1:1000 dilution from stock solution into the ACSF. The inventors used the Axograph software for data acquisition, and Excel (Microsoft) and PRISM (Graphpad) for data analysis.

### Behavioral tests

For conducting similar social behavior experiments, the inventors used group size of 10-15 animals. Animals that had viral expression outside of ILA or rdLS were excluded from analysis. This criterion was pre-established since the inventors wanted to investigate the role of local neurons. The observer was blind to the identity of the mice while performing the behavioral experiments and the subsequent analyses.

*Open arena test.* Mice were introduced into a new arena (60 cm × 60 cm) and allowed to roam freely for 10 min. Using automatic tracking of the test mouse (Any-Maze 7, Stoelting), the inventors quantified the total distance travelled as well as time spent in the surround (20% of the surface) or center (remaining 80% of the surface).

*Elevated plus maze test:* Mice were placed at the center of a maze consisting of a cross with two open arms and two closed arms. They were allowed to explore the maze freely for 5 min. The inventors quantified the amount of time and number of entries in open or closed arms.

*Sociability test.* Test mice were introduced into a same open arena and allowed to roam freely for 10 min. Then, wire cup cages were introduced at opposite corners. One cup had a novel mouse from the same sex and age underneath it. The test mouse was allowed to explore each cup for 5 min. Using automatic tracking of the test mice (ANY-Maze, Stoelting), the inventors quantified the time spent in the area surrounding each cup. Using the interaction times, we calculated a discrimination index for social preference as the following: D! = (time with mouse - time with empty cup) / total interaction time. DI represents the percentage of extra time spent with the mouse compared to the empty cup. The preference exhibited by the test mouse for the mouse compared to the empty cup was used as an index for sociability.

*Social novelty preference test.* This test was performed in the same arena as the open-field and sociability test. Test mouse was allowed to explore the arena for 10 min. Then, the test mouse explored for 5 min two wire cups on opposite corners (learning trial). Each cup contained a novel mouse. Stimulus mice were removed from the cups and the test mouse were placed in an empty cage, the size of its home cage for 30 min. Then, one of the now familiar mice was returned under its cup while a novel mouse was introduced under the other cup (recall trial). The test mouse was free to explore each cup again for 5 min. Importantly, all 3 stimulus mice were littermates housed in the same cage (thus preventing the test mice to use any cage-specific odor clue). The design of this test allows for better exploration of each cup than the classical 3-chamber test while still giving the test mouse freedom to explore the novel or familiar mice unlike the direct interaction test. Using automatic tracking of the test mice (ANY-Maze, Stoelting), the inventors quantified the time the test mice spent in the immediate area surrounding each cup - and therefore, interacting with each stimulus mice - during learning and recall. Using the interaction time from the recall trial, the inventors calculated a discrimination index for social novelty preference as the following: D! = (time with novel - time with familiar) / total interaction time. DI represents the percentage of extra time spent with novel compared to familiar. The preference normally exhibited by the test mouse for the novel compared to the familiar animal was used as an index for social memory. The inventors also calculated the total time spent interacting with stimulus mice during each trial in order to verify that the sociability drive was not affected.

*Novel object preference test:* Same as above but with 3 different small toys of different shape about the size of the mouse. Objects were of the same color.

*Repetitive social presentation test.* Test mice were introduced in a clean cage the same size than their home cage and left to explore for 20 min. Then, a novel mouse of the same age and sex was introduced for four times during 2 min with 10 min inter-trial. For the 5^{th} trial, a novel age- and sex-matched mouse housed in the same cage than the first one was presented. Films were scored offline for social interaction by a trained observer blind to the experimental condition. Sniffing of any part of the body, allo-grooming and close following counted as social interaction. Interaction times were normalized with respect to the first interaction.

*Repetitive object presentation test:* Same as above but with 2 different small toys of different shape about the size of the mouse. Objects were of the same color.

*Food-seeking test:* Mice were food-deprived overnight and then introduce into a large arena (60x60 cm) containing some crushed chow in the middle. The inventors measured the latency to feed and the amount of time spent feeding.

*Food preference test:* Mice were food-deprived overnight and then introduce into a large arena (60x60 cm) containing their regular food (chow) in one corner, and chocolate, as a novel food, in the opposite corner of the arena. The inventors measured the amount of time spent feeding as well as the duration spent in each food zone. Notice that mice are neophobic for food and prefer familiar chow even to more palatable food like chocolate.

*One trial presentation of novel or familiar mice:* Test mice were individually placed in a clean cage the night before the test. The next day a novel mouse or a littermate was introduced for 1 single trial during 2 min. The total time of interaction during the 2 min was simultaneously annotated during film recording. Social interaction was measure by a key-press using Any-Maze when sniffing of any part of the body or chasing occurred. Mice were perfused 1 hour after performing the test and processed for c-fos analysis.

*Kinship preference test:* For this test, the inventors used two females with a positive plug from the same day in order to have two litters of the same developmental stage. After the pups of both litters were born, the inventors started to test the percentage of sibling choice of each pup during consecutive days. From P5 to P14 the test was performed as following: each nest containing their corresponding litter was placed in each corner of the OA. Then, the inventors place each individual pup in a middle point equidistant from both nests. The inventors recorded each pup during 2 min, then they annotated weather the pup ended reaching the sibling or non-sibling nest. Due to the natural increasing exploration behavior of the pups, after P14, the inventors placed all pups of each litter under a pencil cup and placed them in opposite zones of the OA. Then the inventors tested the percentage of sibling preference by measuring the time spent in each zone during 2 min. The inventors only used litters of 5 pups or more, and with similar number of pups between the litters. The inventors also measured the time spent near each nest to calculate a discrimination index for familiar kin similar to the one calculated previously.

*Optogenetic stimulation during social interaction:* Test mice were introduced in a clean cage the same size than their home cage and left to explore for 5 min. Then, 445 nm laser stimulation was applied and a littermate or a novel mouse of the same age and sex was introduced for 2 minutes. Films were scored offline for social interaction by a trained observer blind to the experimental condition. Sniffing of any part of the body, allo-grooming and close following counted as social interaction.

### Fiberphotometry data acquisition and analysis

*Fiber-photometry recording during social or object presentation:* Test mice were individually placed in a clean cage the night before the test. The day of recording, mice were habituated to the optical fiber for three days by placing the fiber on the mouse head and letting it roam free for 15 min in their own cage before the test. Then, the inventors recorded basal activity during two minutes. After 5 minutes, we performed 2 min social or object presentations with 5 minutes in between recordings. Novel and familiar mice or objects were introduced for 1 single trial during 2 min. The order of the familiar or novel presentations were randomized along all the sessions. Movies were annotated online to mark the beginning and end of the interactions.

*Fiber-photometry recording during repetitive social presentation:* Mice were habituated to the optical fiber for three days by placing the fiber on the mouse head and letting it roam free for 10 min. The test was conducted as described previously.

*Optogenetic silencing of CRH-ILA terminals in rdLS and CRH-Biosensor recording in rdLS during interaction with familiar mice:* Mice were injected with the Cre-dependent AAV2/2 CAG.FLEX.ArchT-tdTomato (Addgene #28305 prepared by the University of North Carolina vector core) in the ILA AP: 1.65, ML: ±0.1, DV: -2.6, and a with the CRH-Biosensor pAAV-hSyn-CRF1.0 in LS AP: 0.9, ML: 0, DV: -2.75. For fiber-photometry imaging of LS cells, the inventors implanted the optical ferrule (B280-4419-3, Doric) at the following coordinates AP: 0.9, ML: 0, DV: -2.65. For CRH-ILA terminal silencing in rdLS, the inventors implanted an optical ferrule of 200um using the following coordinates AP: 1.70 ML: 0.2 DV: 3.1, 30° angle. Test mice were introduced in a clean cage the same size as their home cage and left to explore for 15 min. Then, the inventors perform three consecutive trials presenting a littermate for 2 minutes. Trials were developed within 10 min intervals. Light stimulation was developed using the Cobolt-Jive 561nm adjusted at 5mW and applied during Trial 2. CRH-Biosensor activity was recorded using a DORIC system (Basic FMC). Films were scored in real time for social interaction by a trained observer blind to the experimental condition. Sniffing of any part of the body, allo-grooming and close following counted as social interaction.

Fiberphotometry was conducted using a DORIC system (Basic FMC). Two LEDs (405 nm and 465 nm) were coupled to a fluorescence mini-cube (FMC) to deliver light into optical ferrules permanently implanted above the ILA or rdLS. The inventors used 217 Hz as the oscillation frequency for the 465 nm calcium-dependent fluorescence channel, and 319 Hz as the frequency for the 405 nm isosbestic signal. For calcium recording, light was delivered at a final intensity of 2.24 mW (465nm) and 2.76 mW (405 nm) at the tip of the patch-cord before coupling with the implanted ferrule. For the CRF biosensor recording, light was delivered at a final intensity of 1.1 mW (465nm) and 1.9 mW (405 nm) at the tip of the patch-cord in order to limit bleaching. Emitted light between 420 and 450 nm (with 405 nm excitation) and 500 and 540 nm (with 465 nm excitation) were collected through the FMC on separate fiber-coupled Newport 2151 photo-receiver modules. The collected fluorescent signals were collected in AC-low mode and converted to voltage via the formula *V* = *PRG*, where *V* is the collected voltage, *P* is the optical input power in watts, *R* is photodetector responsivity in amps/watts (0.2 - 0.4), and G is the trans-impedance gain of the amplifier. Raw signals for 473 nm excitation (GCaMP6f) and 405 nm excitation (isosbestic signal) were recorded using Doric Neuroscience Studio software.

The inventors used the Guppy software for analysis. After loading the data, the inventors apply an artifact correction criterion consisting on the removal of the first second of the raw data. This allowed them to remove the artifacts that are usually generated right after the light sources turn on. Subtraction of the background fluorescence was calculated via a time-fitted running average of the 465 nm channel relative to the 405 nm control channel using a least squares polynomial fit of degree 1. Δ*F*/*F* is calculated by subtracting the fitted control channel from the signal channel and dividing by the fitted control channel using the formula (465 nm - 405 nm)/405 nm. A peak enveloping Fourier transform was applied to the Δ*F*/*F* signal across the entire trace to identify peaks in activity. Finally, the inventors presented the data as the deviation of the Δ*F*/*F* from its mean (z-score) using the formula (Δ*F*/*F* - *mean of ΔF*/*F*/ *Standard deviation of ΔF*/*F).* For PSTH analysis, the inventors analyzed the z-score for each interaction during the sessions based on the following time window (0 to 3 s after the timestamp).

### Classifier analysis

Fiberphotometry recordings from ILA^{CRH} and rdLS were used to discriminate between novel or familiar interactions. On each session, the inventors detected calcium transients and fit support vector machines (SVM) with linear kernel to discriminate whether each transient occurred during a novel or a familiar interaction. The inventors used two different approaches to fit the SVMs: individual sessions and pseudo-populations^{5,6}. For the individual analysis, the inventors fitted an independent SVM on each individual session (10 sessions for ILA^{CRH} and 10 sessions for rdLS recordings) and evaluated the cross-validated decoding performance per session. Cross-validation was performed by training the classifier on 80% of the dataset and testing the performance on the remaining 20%. Since this partition was random, the inventors repeated this process 20 times and reported the mean. To evaluate the significance of the obtained decoding performance, the inventors built a null-distribution of decoding performances by randomly shuffling the labels for novel or familiar interaction. On each shuffle iteration, the inventors first shuffled the labels and then evaluated the cross-validated performance as in the original dataset (80% train, 20% test, 20 iterations). The inventors repeated this process 1000 times. The reported p-value was obtained by comparing the decoding performance of the original dataset with the null distribution. In all cases, the decoding performance was larger than the largest value of the null distribution (*p* < 0.001). For the pseudo-population analysis, for each session the inventors randomly draw (with replacement) 100 trials from familiar and 100 trials from novel interactions. By combining all recording sessions from a particular brain region, the inventors obtained one matrix for training and one matrix for testing (200 trials x 10 sessions) our linear classifiers (SVM). The matrices for training and testing were obtained by sampling with replacement from 80% and 20% of the trials from the original dataset, respectively. Our procedure ensured that there was no overlap between the train and test matrices. Since the procedure to build these two matrices was random, the inventors repeated this process 100 times and reported the mean decoding performance. To evaluate the significance of the obtained decoding performance for the pseudo-population, the inventors also built a null-distribution of decoding performances by randomly shuffling the labels for novel or familiar interaction on each recording session and proceeded as described above (1000 iterations). The reported p-value was obtained by comparing the decoding performance of the original dataset with the null distribution. As for the individual sessions analysis, the decoding performance was larger than the largest value of the null distribution. The shaded areas correspond to the 2.5 - 97.5 percentile of the null distribution. In all cases, the mean of the null distribution was very close to 50%.

### Statistics

Statistical tests were performed using PRISM (Graphpad). Results presented in the text, figures and figure legends are reported as the mean ± SEM. Unless specified otherwise all tests are two-tailed. * is for *p* < 0.05, ** is for *p* < 0.01, *** is for *p* < 0.001. When multiple observations were done in the same mouse, the inventors used nested statistical tests to take into account the lower degree of freedom.

### RESULTS

### ILA^{CRH} cells project to the rostro-dorsal lateral septum

The inventors injected *CRH-Cre* mice in ILA with a Cre-dependent adeno-associated virus (AAV) expressing membranous GFP and synaptophysin tagged with mRuby in order to visualize axons and synaptic terminals respectively (Figure 1A-B). GFP⁺ fibers in the rostral-dorsal region of the lateral septum were observed (rdLS, Fig. 1C-D). A closer examination confirmed the presence of mRuby-labeled axon terminals in this region (Fig. 1E). Fibers going to posterior LS or other brain regions were not observed (Fig. 2A-C). In addition, fibers in rdLS following injections in nearby mPFC regions were not observed (pre-limbic or anterior cingulate area, Fig. 2D). Next, the retrograde marker CtB-488 in rdLS of *CRH-Cre* mice crossed with a Cre-dependent tdTomato reporter line (CRH-*Cre;Ai9* mice) were injected to visualize CRH⁺ cells (Fig. 1F-G). CtB retrogradely labelled many ILA neurons mostly located in layer 2/3 (Fig. 1H, J). Some CtB⁺ cells co-expressed tdTomato (Fig. 1I) and were mainly found in layer 2/3 of ILA (Fig. 1K). This result was confirmed by injecting a Cre-dependent retrograde monosynaptic herpes simplex virus expressing GFP in the rdLS of *CRH-Cre* mice (Fig. 3A). Consistent with the CtB injections, 79% of CRH/GFP⁺ cells were located in ILA with the rest being located in adjacent regions (Fig. 3B-C). Within ILA, 66% of GFP⁺ cells were located in layer 2/3 (Fig. 3D). Overall, these experiments show that ILA^{CRH} cells from layer 2/3 project to rdLS.

Sections from the same mice for GABA were labelled and observed that 89% of GFP⁺ ILA^{CRH} cells are positive for GABA (Fig. 3E). Furthermore, using *in situ* hybridization markers for excitatory and inhibitory neurons, 92% of ILA^{CRH} cells expressed the mRNA for glutamic acid decarboxylase 2 (*Gad2*) were found while only 3% expressed the mRNA for the vesicular glutamate transporter 1 (*Slc17a7*/*VGlut1*) (Fig. 3G-H), confirming the identity of these neurons as GABAergic. The inventors also patched rdLS neurons in septal slices obtained from CRH-*Cre* mice injected with a Cre-dependent AAV expressing Channelrhodopsin in ILA (Fig. 1L). Stimulation with blue light elicited a large outward current when holding the neurons at + 10 mV (Fig. 1M) No inward currents were detected at -70 mV. The light-induced IPSCs following stimulation of CRH⁺ fibers from ILA were abolished upon application of 2µM SR 95531 and 1µM CGP 55845 which block GABA_{A} and GABA_{B} receptors, respectively (Fig. 1N). Overall, these results show that ILA^{CRH} neurons projecting to rdLS are a subpopulation of GABAergic neurons.

### ILA^{CRH} neurons suppress social interactions with familiar mice and support social novelty preference

Next, the inventors used a chemogenetic approach to modulate the activity of ILA^{CRH} neurons and probe their behavioral function. *CRH-Cre* mice in ILA with Cre-dependent AAVs expressing an inhibitory DREADD (designer receptor exclusively activated by designer drugs) tagged with mCherry (iDREADD) or mCherry only as a control were injected (Fig. 4A-B). Three weeks later, mice were intra-peritoneally injected with the DREADD agonist clozapine N-oxide (CNO, 5 mg/kg) 30 min prior to conducting the behavioral tests. Since a previous study associated CRH⁺ cells in the pre-limbic area of the mPFC with anxiety, the inventors first tested the mice in the open-field to assess locomotion and anxiety (Fig. 5A). Silencing ILA^{CRH} cells had no effect on the distance travelled, the time spent in the center or the surround or the ratio of time spent in the center vs. surround (Fig. 5B-D). Next, the inventors examined the effect of silencing in the elevated plus maze test of anxiety and found no effect on the number of entries or time spent in the open arms relative to the closed ones (Fig. 5E-I). Finally, since glutamatergic cells in the PFC projecting to LS have been reported to be involved in food-seeking behavior, the inventors performed the anxiety-suppressed feeding behavior test, where a food-deprived mouse must venture into the center of an open-field in order to eat (Fig. 5J). Silencing ILA^{CRH} cells had no effect on the latency to feed, the time spent feeding or the number of entries into the food zone (Fig. 5K-M). These controls suggest that ILA^{CRH} cells are functionally distinct neurons without a prominent function in locomotion, anxiety or feeding-related behaviors.

The inventors next tested whether ILA^{CRH} cells regulate social interactions. The mPFC is known to regulate sociability, social preference, social hierarchy as well as emotion discrimination but it remains unclear whether specific sub-regions or populations control different facets of social interactions. First, the inventors silenced ILA^{CRH} cells and assessed the sociability of the mice (preference for a mouse compared to an object, Fig. 6A). Both groups exhibited a strong preference for the mouse compared to the object (Fig. 6B-C). Next, the inventors tested whether ILA^{CRH} cells regulate social novelty preference (Fig. 4C). A subject mouse was exposed to two novel stimulus mice inside wire cup cages in opposite corners of a squared open arena. After 5 min exploring both mice (learning trial), the subject mouse was removed from the arena, placed into an empty housing cage, and one of the two stimulus mice was replaced by a third (novel) mouse. After a 30 min inter-trial interval the subject mouse was reintroduced in the arena (recall trial). Social novelty preference is manifest when the subject mouse spends more time exploring the novel stimulus mouse compared to the familiar one during the recall trial.

The inventors also quantified the preference for exploring the novel mouse by calculating a discrimination index (DI), representing the percentage of extra time the subject mouse spent with the novel compared to the familiar stimulus (see Methods). The inventors measured the social novelty preference of mice in which iDREADD-expressing ILA^{CRH} cells were silenced from the start of the task by injecting CNO systemically 30 min prior to the learning trial. To rule out off-target effects of CNO or of iDREADD expression alone, the inventors examined three control groups of mice: 1. mice injected with CNO expressing mCherry in ILA^{CRH} cells, 2. Mice injected with saline expressing iDREADD in ILA^{CRH} cells; 3. Mice injected with saline expressing mCherry in ILA^{CRH} cells. During recall, the three control groups (mCherry + saline, iDREADD + saline, mCherry + CNO) exhibited a higher interaction time with the novel mouse compared to the familiar one (Fig. 4D), which translated into a high discrimination index preference for the novel mouse (Fig. 4E), indicating intact social novelty preference. However, in the test group in which ILA^{CRH} cells were silenced, the subject mice explored the novel and familiar mice to the same extent (Fig. 4D). As a result, the discrimination index for social novelty preference was not different from zero (Fig. 4E). During learning or recall, the total exploration time of the mice was similar across groups (Fig. 6D-E), suggesting that ILA^{CRH} cell silencing does not affect the motivation to explore. Overall, this experiment shows that ILA^{CRH} cells are necessary for social novelty preference.

During the learning phase of the social novelty preference test, test mice explored each novel conspecific to the same extent than control mice (Fig. 6F), suggesting that silencing ILA^{CRH} cells does not impair social interactions with novel animals. The inventors therefore tested the role of ILA^{CRH} cells during the repetitive social interaction test (also known as the habituation/dishabituation test), where a sex- and age-matched novel mouse is presented 4 times to the test mouse (Fig. 4F). This test offers the advantage of observing the evolution of social interaction with a single novel mouse becoming gradually familiar. Control mice showed a progressive decrease in interaction time with repeated presentations of the mouse (Fig. 4G). When a novel mouse was presented in the final fifth trial, the interaction time jumped back to its initial level, demonstrating that the decreased interaction was not due to fatigue or loss of engagement in the task. In contrast, mice expressing iDREADD showed no decrease in interaction time during the repeated presentations, suggesting that ILA^{CRH} cells are necessary for social familiarization (Fig. 4G). The inventors repeated the experiments injecting saline instead of CNO and both groups exhibited a steady decrease in interaction (Fig. 6G). Next, the inventors asked whether over-activating ILA^{CRH} cells could conversely promote social familiarization and repeated the repetitive social presentation test with mice expressing an excitatory DREADD in ILA^{CRH} neurons. Increasing the activity of ILA^{CRH} cells with CNO slightly facilitated the decrease in social interaction (Fig. 4H), which was not observed when mice were injected with saline (Fig. 6H), indicating that ILA^{CRH} cells can bidirectionally modulate the interaction time with familiar mice. Taken together, these experiments suggest that ILA^{CRH} cells repress social interaction with a familiar mouse and are necessary for social familiarization.

To confirm the chemogenetic approach, CRH-Cre mice expressing excitatory or inhibitory DREADD in ILA were injected with CNO or saline before presenting them with a familiar animal (Fig. 7A). The inventors measured the overlap between c-fos and mCherry expression in ILA (Fig. 7B-C). iDREADD-expressing mice given CNO exhibited less c-fos/mCherry+ cells compared to the saline control, suggesting efficient ILA^{CRH} cells silencing. By contrast, eDREADD-expressing mice injected with CNO showed an increased c-fos/mCherry+ overlap, suggesting ILA^{CRH} cell excitation.

Do ILA^{CRH} cells specifically control social interactions or does it extend to objects as well? The inventors performed tests of novel object recognition, repetitive object presentation and familiar food preference while silencing the ILA^{CRH} and found no effect, indicating that ILA^{CRH} neurons specifically regulate social preferences.

### ILA^{CRH} neurons respond preferentially during familiar social interactions

If ILA^{CRH} cells regulate social interactions with familiar mice, it can be expected the cells to be more active when the mouse interacts with familiar mice than with novel ones. To test this prediction, the inventors performed fiber-photometry of ILA^{CRH} cells. The inventors injected the ILA of CRH-Cre mice with a Cre-dependent AAV expressing the calcium sensor GCaMP6f and implanted an optical ferrule above ILA (Fig. 8A and 9A). Subject mice were presented with novel then familiar mice meanwhile the inventors recorded the calcium activity of the cell population (Fig. 8A-C). First, the inventors calculated the peri-stimulus time histogram using the start of social interaction to synchronize traces (Fig. 8D) and found that familiar mouse presentation elicited a large increase of the calcium response while presentation of a novel mouse elicited a small decrease (Fig. 8E). Then, the inventors automatically detected calcium transients and measured their average amplitude and frequency during each trial. The peaks were higher during familiar compared to novel mouse presentation (Fig. 8F), but the inventors saw no difference in the frequency of events (Fig. 8G). The inventors inverted the order of social presentation and obtained the same results (Fig. 8H). These observations suggest that ILA^{CRH} neuron activity is increased during familiar encounters compared to novel ones.

To confirm whether ILA^{CRH} cell activity differs during novel and familiar mouse presentation, the inventors trained linear classifiers to discriminate between interactions with a novel or familiar mouse using our fiberphotometry recordings. The inventors implemented 2 classifiers using either individual recording sessions (individual) or a meta-session pooling all sessions (pseudo-simultaneous, Fig. 8I). For each classifier, the inventors also computed chance levels using permutation tests (grey bars). Most individual recording sessions yielded a decoding performance above chance with an average 68% accuracy. The pseudo-simultaneous data yielded a decoding performance even higher (79%). These results show that the ILA^{CRH} cell population can code for social familiarity.

The inventors also presented novel and familiar objects and saw no change in activity compared to baseline (Fig. 8J) or between novel and familiar object (Fig. 9B-D). Using the peak amplitudes, the inventors calculated the discrimination indexes (DI) for familiarity preference following object or social presentation (Fig. 8K). DI of social interaction showed a strong preference for social familiarity, unlike the one for object interaction. The inventors then recorded ILA^{CRH} cells during the repetitive social presentation test and found the peak amplitude to increase during familiarization (Fig. 8L). The frequency of events however remained stable (Fig. 8M), similar to what was observed previously (Fig. 8G).

As a further assay of neuronal activity, the inventors measured the expression of the immediate-early gene c-fos. CRH-Cre;Ai9 mice were presented with a novel or familiar mouse for 2 min (Fig. 8N). As expected, mice interacted more with novel than familiar mice (Fig. 8O). Mice were perfused 1 hour later and processed for c-fos immunohistochemistry in order to count the number of ILA^{CRH} neurons expressing c-fos (Fig. 8P). Despite shorter interactions, ILA^{CRH} cells in layer 2/3 exhibited higher c-fos expression following encounters with familiar mice compared to novel (Fig. 8Q), similar to what was already reported for the entire ILA cell population. Indeed, the activation of layer 2/3 ILA^{CRH} cells negatively correlated with the amount of social interaction (Fig. 8R). Overall, these experiments demonstrate that ILA^{CRH} cells are more active during interaction with a familiar mouse than a novel one.

### CRH release in rLS suppresses social interactions with familiar mice to promote social novelty preference

The inventors designed a Cre-dependent shRNA against Crh and expressed it into ILA^{CRH} neurons (Fig. 10A, right). To assess the efficacy of Crh silencing, the inventors quantified Crh and mCherry levels using in situ hybridization (Fig. 10A, bottom). ILA^{CRH} cells expressing the anti-Crh shRNA and mCherry showed a 4-fold decrease in the intensity of Crh labeling compared to nearby non-infected CRH+ cells that did not express mCherry (Fig. 10A-B). No change was seen in cells expressing the scrambled shRNA (Fig. 10A-B). These results indicate that the strategy to reduce Crh level in ILA^{CRH} neurons is both specific and efficient.

Next, the inventors tested CRH-Cre mice expressing scrambled and anti-Crh shRNA during repetitive social presentations. Mice expressing the anti-Crh shRNA in ILA^{CRH} cells showed very little familiarization unlike mice expressing the scrambled shRNA (Fig. 10C). Then, the inventors tested the mice for social novelty preference. Mice expressing anti-Crh shRNA showed no preference during the recall trial (Fig. 10D) and the discrimination index of this group was null (Fig. 10E). Total exploration during learning or recall trials was not different between groups (Fig. 11A-B). Given that ILA^{CRH} neurons co-express GABA, the inventors repeated the same experiments using the shRNA against vGAT mRNA (Fig. 11C-D), which had been used previously to knock-down vGAT in hypothalamic CRH+ neurons50. Unlike knocking-down Crh, knocking-down vGAT expression in ILA^{CRH} cells failed to impair familiarization or social novelty preference (Fig. 11E-H).

Even though the inventors did not observe ILA^{CRH} projections in any region other than rdLS, the inventors sought to validate their previous results using a retrograde targeting approach. The inventors injected a monosynaptic retrograde virus expressing Cre in rdLS (HSV-Cre) and the Cre-dependent viruses expressing anti-Crh shRNA or scrambled shRNA in the ILA of WT mice (Fig. 11I). Removing Crh from rdLS-projecting cells in the ILA impaired familiarization and social novelty preference (Fig. 11J-N), similar to previous results.

The inventors then asked whether CRH release from ILA^{CRH} in rLS cells was necessary to mediate familiarization and social novelty preference. First, the inventors sought to confirm that CRH release in LS occurs preferentially during familiar social interaction and leveraged the recently developed CRH biosensor CRF1.051. WT mice were injected in rdLS with an AAV expressing CRF1.0 and an optical ferrule was implanted above it (Fig. 10F). The inventors presented novel and familiar mice in a random order meanwhile recording CRH activity events (Fig. 10H). Presentation of a familiar mouse induced responses of larger amplitude compared to the presentation of a novel mouse (Fig. 10I), despite the mice interacting less (Fig. 10G). There was no change in the frequency of events (Fig. 10J). The discrimination index based on z-score peak amplitude showed a significant preference for the response for familiar mice (Fig. 10K). This experiment demonstrates that CRH release in rLS is higher during familiar social interactions compared to novel social interactions.

Next, the inventors used optogenetics to silence ILA^{CRH} cell terminals in rdLS. CRH-Cre mice were injected in ILA with Cre-dependent AAVs expressing Archaerhodopsin tagged with tdTomato (Arch) or tdTomato only as a control and an optical ferrule was implanted above rdLS (Fig. 10L). Light from a 561 nm laser was applied continuously during either the learning or recall trials of the social novelty preference test. When light was applied during the learning trial, both groups exhibited social novelty preference during the recall trial. However, when light was applied during the recall trial, mice expressing Arch failed to show a preference for the novel mouse while mice expressing tdTomato only did (Fig. 10M-N). All groups showed the same extent of total interaction during learning and recall (Fig. 11O-P). The inventors also tested the mice during repetitive social presentations and applied the light stimulation during trials 2-4 or no light. Arch-expressing mice with light failed to familiarize to the novel mouse unlike tdTomato only -expressing mice with light or Arch-expressing mice with no light (Fig. 10O).

The inventors tested the efficiency of our terminal-silencing approach combining Arch-mediated silencing and CRH recording. CRH-Cre mice were injected with a Cre-dependent virus expressing Arch in the ILA and a virus expressing the biosensor CRF1.0 in rdLS (Fig. 12A-B). Two optical ferrules were implanted above rdLS. Then, the inventors exposed the mice to a familiar mouse for 3 sessions of 2 min separated by 10 min intervals. The 561 nm laser was turned on only during the middle presentation in order to stimulate Arch in ILACRH terminals in rdLS (Fig. 12C). Activating Arch efficiently decreased the frequency of CRH-related transients (Fig. 12D). The amplitude of the transients followed a similar trend (Fig. 12E). Importantly, turning on the laser increased the amount of social interaction with the familiar mouse, suggesting that ILA^{CRH} fibers regulate the amount of social interaction with familiar mice (Fig. 12F). Taken together, these experiments show that CRH release from ILA^{CRH} cells in rdLS during social encounters suppress social interactions with familiar mice to promote social novelty preference.

### CRHR1 activation in rdLS suppresses social interaction with familiar mice and support social novelty preference

The inventors injected CRHR1-Cre mice with a Cre-dependent AAV expressing GFP and labelled several neurons in rdLS (Fig. 13A), the same region targeted by terminals of ILA^{CRH} cells (Fig. 1C-D). Then, the inventors crossed the CRHR1-Cre line with the Ai9 tdTomato reporter in order to visualize CRHR1+ neurons, prepared acute LS slices and obtained whole-cell patch-clamp recordings from rdLSCRHR1 neurons clamped at -60 mV. Application of 300 nM stressin-1, a CRHR1 agonist, induced an inward current (Fig. 13B). This experiment suggests that CRH release in rdLS depolarizes CRHR1+ neurons.

Then, the inventors implanted mice with a cannula in rdLS and infused them with the CRHR1 antagonist antalarmin diluted in DMSO or DMSO only before running them for the social novelty preference test (Fig. 13C). Mice infused with DMSO exhibited normal preference for the novel mouse whereas mice infused with antalarmin showed no novelty preference (Fig. 13C-D). Total exploration time during learning or recall was not different between groups (Fig. 14A-B).

Next, the inventors performed chemogenetic silencing of CRHR1+ neurons in rdLS. The inventors injected CRHR1-Cre mice with a Cre-dependent AAVs expressing inhibitory DREADD tagged with mCherry or mCherry only (Fig. 13E). Then, the inventors tested the mice for social novelty preference and observed a preference for the novel mouse in the 3 control groups but not in the test group (Fig. 13F). Consequently, the discrimination index of the test group was not different from 0 unlike the ones of the control groups (Fig. 13G). The total interaction times during learning or recall or the interaction time with each novel mouse during learning were similar across all groups (Fig. 14C-E). The inventors also ran the mice for the repetitive social presentation test and observed no familiarization in the test group (Fig. 13H). Overall, these experiment shows that activation of the CRHR1 receptor in rdLS is necessary for familiarization and social novelty preference.

Then, the inventors tested whether CRHR1+ neurons in rdLS were preferentially activated during familiar encounters compared to novel ones. The inventors presented CRHR1-Cre;Ai9 mice with either novel or familiar mice before perfusing them and labeling for c-fos (Fig. 13!). The percentage of rdLSCRHR1 neurons expressing c-fos during familiar interaction was 3-fold higher than the one during novel interaction (Fig. 13J). Overall, these experiments demonstrate that rdLSCRHR1 neurons are preferentially recruited during familiar encounters in order to regulate familiarization and social novelty preference.

### CRHR1 activation disinhibits rLS to suppress social interactions with familiar mice

The inventors prepared acute LS slices from WT mice and recorded spontaneous inhibitory post-synaptic currents (IPSCs) from rdLS cells in whole-cell configuration before applying the CRHR1 agonist stressin-1 (300 nM, Fig. 15A-B). Stressin-1 application for 15 min decreased the frequency and integrated charge of spontaneous IPSCs (Fig. 15B-C and E). IPSC amplitude also exhibited a trend toward a decrease (Fig. 15D). This effect was not seen when rLS neurons were recorded for 15 min without application of the agonist (Fig. 16 A1-A5). In addition, application of stressin-1 while recording from vLS neurons in posterior LS slices had no effect (Fig. 16 B1-B6), suggesting that the agonist effect was not generalized to the entire LS, consistent with the pattern of CRHR1 expression. Taken together, these results suggests that CRH release disinhibits rLS neurons.

The inventors thought of eliciting CRH release in rdLS from ILA fibers since CRH could originate from other regions. The inventors expressed channel rhodopsin in ILA^{CRH} neurons and prepared rdLS slices (Fig. 15F). The inventors obtained whole-cell recordings of rdLS neurons and recorded spontaneous inhibitory events (sIPSC) before and after applying a tetanic light stimulation. Light stimulation induced a decrease in sIPSC frequency, amplitude and charge (Fig. 15G-I), similar to stressin-1 application. In addition, the inventors applied a local electrical stimulation to induce a large elicited IPSC. The amplitude of which was also decreased following light stimulation (Fig. 15J). The inventors repeated these recordings with 300 nM antalarmin in the bath to confirm that the decrease in inhibition following light stimulation was CRH-dependent. Indeed, antalarmin application blocked the disinhibition (Fig. 15G-J). Taken together these results demonstrate that CRH release from ILA disinhibits rLS neurons.

The inventors recorded responses of rLS neurons during interaction with a novel or familiar mouse using fiber-photometry. C57BU6J wild-type mice were injected in rLS with an AAV expressing GCaMP6f and an optical ferrule was implanted above it (Fig. 15K and 17A-C). The inventors presented novel and familiar mice and measured the interaction time, average peak amplitude and peak frequency (Fig. 15L-N). Similar to our recordings of ILA^{CRH} neurons, presentation of a familiar mouse induced transients of larger amplitude compared to the presentation of a novel mouse (Fig. 15M), despite the mice interacting less (Fig. 15L). There was no change in the frequency of transients however (Fig. 15N). The discrimination index based on the peak amplitude showed a significant preference in response to a familiar mouse (Fig. 15O). To determine further whether rLS activity alone can differentiate between novel and familiar mouse presentation, the inventors trained linear classifiers to discriminate between interactions with a novel or familiar mouse based on our fiberphotometry recordings. The inventors implemented 2 classifiers using either individual recording sessions (individual) or creating a meta-session pooling all sessions (pseudo-simultaneous, Fig. 15P). For each classifier, the inventors also computed chance levels using permutation tests (grey bars). Most individual recording session yielded a decoding performance above chance with an average of 59% accuracy while the pseudo-simultaneous data yielded a decoding performance even higher (81%). This shows that rLS neurons can encode for social familiarity. The inventors also measure rLS activity during the repetitive social presentation test and observed that the peak amplitude of calcium events increased from trial 1 to trial 3 when familiarization is taking place (Fig. 17D-E). Similar to the percentage of c-fos+ L2/3 ILA^{CRH} cells, the calcium activity anti-correlated with the amount of social interaction (Fig. 17F).

The inventors examined c-fos expression in LS following novel or familiar social encounters (Fig. 15Q) in the same cohort of mice than the one used to look at c-fos expression in ILA. rLS responded preferentially to familiar mouse presentation (Fig. 15R-S), similar to layer 2/3 ILA^{CRH} neurons. C-fos expression was specifically upregulated in a spatially defined band of rLS cells bordering the lateral ventricle (Fig. 6R, right) while exposure to a novel mouse failed to activate the same population (Fig. 15R, left). Taken together, the fiberphotometry recordings and c-fos labelling demonstrate that a population of rLS neurons is activated preferentially during a familiar encounter compared to a novel one. Similar to L2/3 ILA^{CRH} neurons, activation of rLS neurons tended to correlate negatively with the amount of social interaction (Fig. 18A). Interestingly, activation of layer 2/3 ILA^{CRH} cells plotted against rdLS activation demonstrated a strong positive correlation, suggesting that one population might control the other (Fig. 15T). The inventors also quantified c-fos expression in posterior dorsal LS (dLS) and posterior ventral LS (vLS) and observed no preferential response to familiar presentation compared to novel nor correlation with the amount of interaction (Fig. 18B-G).

### CRH release in rdLS disinhibits rLS to suppresses social interactions with familiar mice.

Does the activation of rLS during familiar encounters depends on CRH release from ILA^{CRH} cells? The inventors tested this hypothesis by measuring c-fos expression in mice where Crh was knocked down in ILA. CRH-Cre mice were injected in ILA with Cre-dependent AAVs expressing anti-Crh shRNA or a scrambled shRNA control. Mice were then presented with a familiar littermate and sections containing the ILA and rLS were labeled for c-fos (Fig. 19A-C). Importantly, mice expressing the anti-Crh shRNA interacted more with a familiar mouse than control mice, suggesting that Crh reduces social interaction with a familiar mouse (Fig. 19D). Loss of Crh did not alter c-fos expression in layer 2/3 ILA^{CRH} cells (Fig. 19E) but reduced the density of c-fos expression in rLS (Fig. 19F). As previously, control mice exhibited a correlation between c-fos levels in layer 2/3 ILA^{CRH} and rLS neurons but mice depleted of Crh in ILA did not (Fig. 19G).

The inventors next tested whether CRHR1+ cells in rdLS controls the disinhibition of rdLS following familiar social encounter. The inventors expressed inhibitory DREADD in rdLSCRHR1 neurons and injected the mice with CNO before presenting them with a familiar mouse (Fig. 19H-I). The inventors then perfused the mice and labeled rLS slices against c-fos (Fig. 19J-K). Silencing rdLSCRHR1 neurons decreased the density of c-fos labeled cells in rdLS (Fig. 19L) while increasing the amount of social interaction (Fig. 19I). Taken together, these experiments demonstrate that CRH release from ILA and activation of rdLSCRHR1 neurons during familiar encounters disinhibits a specific population of rLS cells bordering the lateral ventricles and suppress social interactions with a familiar mouse.

The findings suggest that rLS disinhibition suppresses social interactions. To explore this further, the inventors examined the effects of optogenetic activation of rLS neurons. The inventors injected an AAV expressing Channelrhodopsin (ChR) tagged with mCherry or mCherry only in rLS of C57BL/6J wild-type mice and implanted an optical ferrule above it (Fig. 19M-N). The inventors then presented a familiar mouse for 2 min while stimulating ChR using a 445 nm laser (1 ms stimulation at 20 Hz) and measured the interaction time as well as the mean duration of each interaction bout. As an additional control, the inventors measured behavior in ChR-expressing mice without laser stimulation. Activation of rLS neurons decreased the amount of social interaction with familiar mice (Fig. 19O) due to shorter interaction bouts each time the mice met (Fig. 19P) without affecting locomotion (Fig. 19Q). These optogenetic experiments demonstrate that rLS is able to decrease social interactions. Altogether the study shows that ILA^{CRH} cells are activated during familiar mouse encounters, leading to release of CRH in rdLS causing its disinhibition. Disinhibition of rLS in turn suppress social interaction which leads to the decrease in social interaction observed during familiarization. Inhibition of social interaction with familiar mice promotes social novelty preference when novel and familiar mice are presented simultaneously.

### Increased CRH expression in ILA supports a shift in social preference in young mice

The inventors tested when the shift in social preference occurs in mice by giving young C57BU6J wild-type mice the choice to interact with their familiar siblings or with unfamiliar non-siblings every day from P7 to P21. Between P7 and P15, mice preferred to interact with their siblings (Fig. 20A) and their discrimination index was strongly skewed toward familiarity (Fig. 20B). Preference then gradually shifted toward novel mice (Fig. 20A) and the index leaned toward social novelty after P16 (Fig. 20B).

Both the PFC and LS have been shown to control social novelty preference and the LS is also involved in the preference young rats display for their own (familiar) kin compared to non-kin3. The inventors then asked when ILA neurons begin to express CRH and whether the emergence of the ILA to rdLS circuit described above contributes to the shift in social preference. The inventors counted CRH-tdTomato+ cells at P7, P15 and P21 in CRH-Cre;Ai9 mice (Fig. 20C-D) and observed a strong increase in the density of ILA^{CRH} cells from P7 to P21 (Fig. 20E). Interestingly, the increase was the strongest in ILA compared to other prefrontal regions (Fig. 20F). Within ILA, the increase of CRH+ cells proceeded mostly from an increase in CRH+ cells located in layer 2/3 (Fig. 20G), which is the layer containing ILA^{CRH} cells projecting to rdLS. Comparing the number of ILA^{CRH} cells per section to the retrogradely labeled ones, suggests that at least 60% of these cells project to rdLS. Closer inspection of CRH+ cells in PLA and ACA revealed less or no increase in layer 2/3 (Fig. 21A-B). *In situ* hybridization against Crh in C57BL/6 WT mice show a similar increase in Crh+ neuron density in mPFC, PLA and ILA (Fig. 21C-F). Overall, these experiments demonstrate that the strengthening of the ILA^{CRH} to rdLS circuit correlates with the shift from social familiarity to social novelty preference in young pups.

The inventors next probed whether the emergence of the circuit causes the shift in preference. P5 pups were injected with AAVs expressing anti-Crh or scrambled shRNAs. The inventors reasoned the AAVs would be taken up by many ILA neurons so that, as soon as CRH expression begins, so would the Cre recombinase expression and therefore shRNA expression under the control of the fast-expressing U6 promoter. 2 days after the injection, the inventors began testing the injected pups for social preference and observed preference shifted at P14 in the control group (Fig. 20H), similar to our previous experiment on wild-type mice (Fig. 20A). The test group lacking Crh however continued to exhibit familiar preference until P20 (Fig. 20H). Consistently, discrimination index for the control group inverted before and after P16 reflecting the shift in social preference while the index for the Crh-depleted group remained oriented toward familiar choice (Fig. 20I). The inventors performed *in situ* hybridization against Crh and mCherry at the end of the behavioral testing (P21) in order to verify the efficacy of the shRNA-mediated Crh depletion technique in young pups (Fig. 21G-H). Similar to the results in adults, cells expressing the anti-Crh shRNA exhibited a strong decrease in Crh labeling intensity (Fig. 21I). Overall, these experiments suggest that increased CRH expression in ILA is responsible for the shift in social preference displayed by young mice.

## Claims

1. A CRHR1 agonist for use in the treatment and/or prevention of social anxiety disorders.

2. CRHR1 agonist for use according to claim 1, wherein the social anxiety disorder is separation anxiety disorder or avoidant personality disorder.

3. CRHR1 agonist for use according to claim 1 or 2, wherein the CRHR1 agonist is selected from the list consisting of: corticotropin-releasing hormone (CRH) or corticotropin-releasing factor (CRF), Urocortin 1, Sauvagine, Urocortin 2, human TyrO-CRF and Stressin 1.

4. CRHR1 agonist for use according to any of claims 1 to 3, wherein the CRHR1 agonist is corticotropin-releasing hormone (CRH), preferably ovine CRH, human CRH, mouse CRH or rat CRH.

5. CRHR1 agonist for use according to claim 4, wherein the CRH comprises or consists of the amino acid sequence SEQ ID NO: 1.

6. CRHR1 agonist for use according to any of claims 1 to 5, wherein the CRHR1 agonist is comprised in a composition, preferably a pharmaceutical composition.

7. CRHR1 agonist for use according to claim 6, wherein the pharmaceutical composition comprises a pharmaceutically acceptable carrier and/or an excipient.

8. CRHR1 agonist for use according to any of claims 1 to 7, wherein the CRHR1 agonist or the composition is formulated for subcutaneous or intranasal administration.

9. CRHR1 agonist for use according to any of claims 1 to 8, wherein the therapeutically effective amount of CRHR1 agonist or the composition comprises CRHR1 agonist is a cumulative doses less than 300 µg/h, preferably less than 200 µg/h.

10. CRHR1 agonist for use according to any of claims 1 to 8, wherein the therapeutically effective amount of CRHR1 agonist or the composition comprises CRHR1 agonist for subcutaneous or intranasal application is between 1 and 30 µg/kg body weight.
